# EUROPEAN PATENT APPLICATION

(11) **EP 1 394 256 A2**
(43) Date of publication of application: **03.03.2004**
(21) Application number: 03013389.6
(22) Date of filing: 08.05.1992
(51) Int. Cl.: C12N 15/51

(54) **HCV genomic sequences for diagnostics and therapeutics**

(30) Priority: 08.05.1991 US 697326
(62) Divisional of application: 92913881.6
(71) Applicant: Chiron Corporation, Emeryville, CA 94622-8097 (US)
(72) Inventor: Cha, Tai-An, San Ramon, CA 94583 (US); Beall, Eileen, Walnut Creek, CA 94596 (US); Irvine, Bruce, Concord, CA 94519 (US); Kolberg, Janice, Hercules, CA 94547 (US); Urdena, Michael S., Alamo, CA 94501 (US)
(74) Representative: UEXKÜLL & STOLBERG

(57) **Abstract**

The present application features nucleic acid, peptide an antibody compositions relating to genotypes of hepatitis C virus and methods of using such compositions for diagnostic and therapeutic purposes.

SEQUENCE LISTING

(1) GENERAL INFORMATION:

(i) APPLICANT: Tai-An Cha

(ii) TITLE OF INVENTION: HCV GENOMIC SEQUENCES
FOR DIAGNOSTICS AND THERAPEUTICS

(iii) NUMBER OF SEQUENCES: 147

(iv) CORRESPONDENCE ADDRESS:
(A) ADDRESSEE: Wolf, Greenfield & Sacks, P.C.
(B) STREET: 600 Atlantic Avenue
(C) CITY: Boston
(D) STATE: Massachusetts
(E) COUNTRY: USA
(F) ZIP: 02210

(v) COMPUTER READABLE FORM:
(A) MEDIUM TYPE: Diskette, 5.25 inch
(B) COMPUTER: IBM compatible
(C) OPERATING SYSTEM: MS-DOS Version 3.3
(D) SOFTWARE: WordPerfect 5.1

(vi) CURRENT APPLICATION DATA:
(A) APPLICATION NUMBER: Not Available
(B) FILING DATE: Not Available
(C) CLASSIFICATION: Not Available

(vii) PRIOR APPLICATION DATA:
(A) APPLICATION NUMBER: 07/697,326
(B) FILING DATE: 8 May 1991

(viii) ATTORNEY/AGENT INFORMATION:
(A) NAME: Janiuk, Anthony J.
(B) REGISTRATION NUMBER: 29,809
(C) REFERENCE/DOCKET NUMBER: C0772/7000

(ix) TELECOMMUNICATION INFORMATION:
(A) TELEPHONE: (617) 720-3500
(B) TELEFAX: (617) 720-2441
(C) TELEX: EZEKIEL

(2) INFORMATION FOR SEQ ID NO: 1:

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE: (ATCC # 40394)
(C) INDIVIDUAL ISOLATE: ns5hcv1

(xi) SEQUENCE DESCRIPTION: SEQ ID NO: 1
CTCCACAGTC ACTGAGAGCG ACATCCGTAC GGAGGAGGCA 40
ATCTACCAAT GTTGTGACCT CGACCCCCAA GCCCGCGTGG 80
CCATCAAGTC CCTCACCGAG AGGCTTTATG TTGGGGGCCC 120
TCTTACCAAT TCAAGGGGGG AGAACTGCGG CTATCGCAGG 160
TGCCGCGCGA GCGGCGTACT GACAACTAGC TGTGGTAACA 200
CCCTCACTTG CTACATCAAG GCCCGGGCAG CCTGTCGAGC 240
CGCAGGGCTC CAGGACTGCA CCATGCTCGT GTGTGGCGAC 280
GACTTAGTCG TTATCTGTGA AAGCGCGGGG GTCCAGGAGG 320
ACGCGGCGAG CCTGAGAGCC 340

(2) INFORMATION FOR SEQ ID NO: 2:

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5i21

(xi) SEQUENCE DESCRIPTION: SEQ ID NO: 2
CTCCACAGTC ACTGAGAGCG ACATCCGTAC GGAGGAGGCA 40
ATTTACCAAT GTTGTGACCT GGACCCCCAA GCCCGCATGG 80
CCATCAAGTC CCTCACTGAG AGGCTTTATG TCGGGGGCCC 120
TCTTACCAAT TCAAGGGGGG AGAACTGCGG CTACCGCAGG 160
TGCCGCGCGA GCGGCGTACT GACAACTAGC TGTGGTAACA 200
CCCTCACTTG CTACATCAAG GCCCGGGCAG CCTGTCGAGC 240
CGCAGGGCTC CAGGACTGCA CCATGCTTGT GTGTGGCGAC 280
GACTTAGTCG TTATCTGTGA AAGTGCGGGG GTCCAGGAGG 320
ACGCGGCGAG CCTGAGAGCC 340

(2) INFORMATION FOR SEQ ID NO: 3:

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) individual isolate: ns5pt1

(xi) SEQUENCE DESCRIPTION: SEQ ID NO: 3
CTCCACAGTC ACTGAGAGCG ACATCCGTAC GGAGGAGGCA 40
ATCTACCAAT GTTGTGATCT GGACCCCCAA GCCCGCGTGG 80
CCATCAAGTC CCTCACTGAG AGGCTTTACG TTGGGGGCCC 120
TCTTACCAAT TCAAGGGGGG AGAACTGCGG CTACCGCAGG 160
TGCCGGGCGA GCGGCGTACT GACAACTAGC TGTGGTAATA 200
CCCTCACTTG CTACATCAAG GCCCGGGCAG CCTGTCGAGC 240
CGCAGGGCTC CGGGACTGCA CCATGCTCGT GTGTGGTGAC 280
GACTTGGTCG TTATCTGTGA GAGTGCGGGG GTCCAGGAGG 320
ACGCGGCGAG CCTGAGAGCC 340

(2) INFORMATION FOR SEQ ID NO: 4

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5gm2

(xi) SEQUENCE DESCRIPTION: SEQ ID NO: 4
CTCTACAGTC ACTGAGAACG ACATCCGTAC GGAGGAGGCA 40
ATTTACCAAT GTTGTGACCT GGACCCCCAA GCCCGCGTGG 80

CCATCAAGTC CCTCACTGAG AGGCTTTATG TTGGGGGCCC 120
CCTTACCAAT TCAAGGGGGG AAAACTGCGG CTATCGCAGG 160
TGCCGCGCGA GCGGCGTACT GACAACTAGC TGTGGTAACA 200
CCCTCACTTG CTACATTAAG GCCCGGGCAG CCTGTCGAGC 240
CGCAGGGCTC CAGGACTGCA CCATGCTCGT GTGTGGCGAC 280
GACTTAGTCG TTATCTGTGA GAGTGCGGGA GTCCAGGAGG 320
ACGCGGCGAA CTTGAGAGCC 340

(2) INFORMATION FOR SEQ ID NO: 5

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5us17

(xi) SEQUENCE DESCRIPTION: SEQ ID NO: 5
CTCCACAGTC ACTGAGAGCG ATATCCGTAC GGAGGAGGCA 40
ATCTACCAGT GTTGTGACCT GGACCCCCAA GCCCGCGTGG 80
CCATCAAGTC CCTCACCGAG AGGCTTTATG TCGGGGGCCC 120
TCTTACCAAT TCAAGGGGGG AAAACTGCGG CTATCGCAGG 160
TGCCGCGCAA GCGGCGTACT GACAACTAGC TGTGGTAACA 200

CCCTCACTTG TTACATCAAG GCCCAAGCAG CCTGTCGAGC 240
CGCAGGGCTC CGGGACTGCA CCATGCTCGT GTGTGGCGAC 280
GACTTAGTCG TTATCTGTGA AAGTCAGGGA GTCCAGGAGG 320
ATGCAGCGAA CCTGAGAGCC 340

(2) INFORMATION FOR SEQ ID NO: 6

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5sp2

(xi) SEQUENCE DESCRIPTION: SEQ ID NO: 6
CTCTACAGTC ACTGAGAGCG ATATCCGTAC GGAGGAGGCA 40
ATCTACCAAT GTTGTGACCT GGACCCCGAA GCCCGTGTGG 80
CCATCAAGTC CCTCACTGAG AGGCTTTATG TTGGGGGCCC 120
TCTTACCAAT TCAAGGGGGG AGAACTGCGG CTACCGCAGG 160
TGCCGCGCAA GCGGCGTACT GACGACTAGC TGTGGTAATA 200
CCCTCACTTG TTACATCAAG GCCCGGGCAG CCTGTCGAGC 240
CGCAGGGCTC CAGGACTGCA CCATGCTCGT GTGTGGCGAC 280

GACCTAGTCG TTATCTGCGA AAGTGCGGGG GTCCAGGAGG 320
ACGCGGCGAG CCTGAGAGCC 340

(2) INFORMATION FOR SEQ ID NO: 7

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5j1

(xi) SEQUENCE DESCRIPTION: SEQ ID NO: 7
CTCCACAGTC ACTGAGAATG ACACCCGTGT TGAGGAGTCA 40
ATTTACCAAT GTTGTGACTT GGCCCCCGAA GCCAGACAGG 80
CCATAAGGTC GCTCACAGAG CGGCTCTATG TCGGGGGTCC 120
TATGACTAAC TCCAAAGGGC AGAACTGCGG CTATCGCCGG 160
TGCCGCGCGA GCGGCGTGCT GACGACTAGC TGCGGTAATA 200
CCCTCACATG CTACCTGAAG GCCACAGCGG CCTGTCGAGC 240
TGCCAAGCTC CAGGACTGCA CGATGCTCGT GAACGGAGAC 280
GACCTTGTCG TTATCTGTGA AAGCGCGGGG AACCAAGAGG 320
ACGCGGCAAG CCTACGAGCC 340

(2) INFORMATION FOR SEQ ID NO: 8

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5k1

(xi) SEQUENCE DESCRIPTION: SEQ ID NO: 8
CTCAACGGTC ACTGAGAATG ACATCCGTGT TGAGGAGTCA 40
ATTTACCAAA GTTGTGACTT GGCCCCCGAG GCCAGACAAG 80
CCATAAGGTC GCTCACAGAG CGGCTTTACA TCGGGGGCCC 120
CCTGACTAAT TCAAAAGGGC AGAACTGCGG CTATCGCCGA 160
TGCCGCGCCA GCGGTGTGCT GACGACTAGC TGCGGTAATA 200
CCCTCACATG TTACTTGAAG GCCACTGCGG CCTGTAGAGC 240
TGCGAAGCTC CAGGACTGCA CGATGCTCGT GTGCGGAGAC 280
GACCTTGTCG TTATCTGTGA AAGCGCGGGA ACCCAGGAGG 320
ATGCGGCGAG CCTACGAGTC 340

(2) INFORMATION FOR SEQ ID NO: 9

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5k1.1

(xi) SEQUENCE DESCRIPTION: SEQ ID NO: 9
CTCAACGGTC ACCGAGAATG ACATCCGTGT TGAGGAGTCA 40
ATTTATCAAT GTTGTGCCTT GGCCCCCGAG GCTAGACAGG 80
CCATAAGGTC GCTCACAGAG CGGCTTTATA TCGGGGGCCC 120
CCTGACCAAT TCAAAGGGGC AGAACTGCGG TTATCGCCGG 160
TGCCGCGCCA GCGGCGTACT GACGACCAGC TGCGGTAATA 200
CCCTTACATG TTACTTGAAG GCCTCTGCAG CCTGTCGAGC 240
CGCGAAGCTC CAGGACTGCA CGATGCTCGT GTGTGGGGAC 280
GACCTTGTCG TTATCTGTGA AAGCGCGGGA ACCCAGGAGG 320
ACGCGGCGAA CCTACGAGTC 340

(2) INFORMATION FOR SEQ ID NO: 10

(i) SEQUENCE CHARACTERISTICS:
(A) LENGTH: 340 nucleotides
(B) TYPE: nucleic acid

(C)   STRANDEDNESS:  single
(D)   TOPOLOGY: linear

(ii)   MOLECULE TYPE:  DNA

(vi)   ORIGINAL SOURCE:
(C)   INDIVIDUAL ISOLATE:  ns5gh6

(xi)   SEQUENCE DESCRIPTION:  SEQ ID NO:  10
CTCAACGGTC ACTGAGAGTG ACATCCGTGT CGAGGAGTCG    40
ATTTACCAAT GTTGTGACTT GGCCCCCGAA GCCAGGCAGG    80
CCATAAGGTC GCTCACCGAG CGACTTTATA TCGGGGGCCC   120
CCTGACTAAT TCAAAAGGGC AGAACTGCGG TTATCGCCGG   160
TGCCGCGCGA GCGGCGTGCT GACGACTAGC TGCGGTAATA   200
CCCTCACATG TTACTTGAAG GCCTCTGCAG CCTGTCGAGC   240
TGCAAAGCTC CAGGACTGCA CGATGCTCGT GAACGGGGAC   280
GACCTTGTCG TTATCTGCGA GAGCGCGGGA ACCCAAGAGG   320
ACGCGGCGAG CCTACGAGTC                         340

(2)  INFORMATION FOR SEQ ID NO: 11

(i)   SEQUENCE CHARACTERISTICS:
(A)   LENGTH: 340 nucleotides
(B)   TYPE: nucleic acid
(C)   STRANDEDNESS:  single
(D)   TOPOLOGY: linear

(ii) MOLECULE TYPE: DNA

(vi) ORIGINAL SOURCE:
(C) INDIVIDUAL ISOLATE: ns5sp1

(xi) SEQUENCE DESCRIPTION: SEQ ID NO: 11
CTCCACAGTC ACTGAGAGTG ACATCCGTGT TGAGGAGTCA 40
ATTTACCAAT GTTGTGACTT GGCCCCCGAA GCCAGACAGG 80
CTATAAGGTC GCTCACAGAG CGGCTGTACA TCGGGGGTCC 120
CCTGACTAAT TCAAAAGGGC AGAACTGCGG CTATCGCCGG 160
TGCCGCGCAA GCGGCGTGCT GACGACTAGC TGCGGTAACA 200
CCCTCACATG TTACTTGAAG GCCTCTGCGG CCTGTCGAGC 240
TGCGAAGCTC CAGGACTGCA CGATGCTCGT GTGCGGTGAC 280
GACCTTGTCG TTATCTGTGA GAGCGCGGGA ACCCAAGAGG 320
ACGCGGCGAG CCTACGAGTC 340

(2) INFORMATION FOR SEQ ID NO: 12

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:

(C)   individual isolate:  ns5sp3

(xi) SEQUENCE DESCRIPTION: SEQ ID NO: 12
CTCAACAGTC ACTGAGAGTG ACATCCGTGT TGAGGAGTCA    40
ATCTACCAAT GTTGTGACTT GGCCCCCGAA GCCAGACAGG    80
CTATAAGGTC GCTCACAGAG CGGCTTTACA TCGGGGGTCC   120
CCTGACTAAT TCAAAAGGGC AGAACTGCGG CTATCGCCGG   160
TGCCGCGCAA GCGGCGTGCT GACGACTAGC TGCGGTAATA   200
CCCTCACATG TTACCTGAAG GCCAGTGCGG CCTGTCGAGC   240
TGCGAAGCTC CAGGACTGCA CAATGCTCGT GTGCGGTGAC   280
GACCTTGTCG TTATCTGTGA GAGCGCGGGG ACCCAAGAGG   320
ACGCGGCGAG CCTACGAGTC                         340

(2) INFORMATION FOR SEQ ID NO: 13

(i)    SEQUENCE CHARACTERISTICS:
       (A)   LENGTH: 340 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:
       (C)   INDIVIDUAL ISOLATE:  ns5k2

(xi) SEQUENCE DESCRIPTION: SEQ ID NO: 13

CTCAACCGTC ACTGAGAGAG ACATCAGAAC TGAGGAGTCC 40
ATATACCGAG CCTGCTCCCT GCCTGAGGAG GCTCACATTG 80
CCATACACTC GCTGACTGAG AGGCTCTACG TGGGAGGGCC 120
CATGTTCAAC AGCAAGGGCC AGACCTGCGG GTACAGGCGT 160
TGCCGCGCCA GCGGGGTGCT CACCACTAGC ATGGGGAACA 200
CCATCACATG CTATGTAAAA GCCCTAGCGG CTTGCAAGGC 240
TGCAGGGATA GTTGCACCCT CAATGCTGGT ATGCGGCGAC 280
GACTTAGTTG TCATCTCAGA AAGCCAGGGG ACTGAGGAGG 320
ACGAGCGGAA CCTGAGAGCT 340

(2) INFORMATION FOR SEQ ID NO: 14

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5arg8

(xi) SEQUENCE DESCRIPTION: SEQ ID NO: 14
CTCTACAGTC ACGTAAAAGG ACATCACATC CTAGGAGTCC 40
ATCTACCAGT CCTGTTCACT GCCCGAGGAG GCTCGAACTG 80
CTATACACTC ACTGACTGAG AGACTATACG TAGGGGGGCC 120

CATGACAAAC AGCAAGGGCC AATCCTGCGG GTACAGGCGT 160
TGCCGCGCGA GCGCAGTGCT CACCACCAGC ATGGGCAACA 200
CACTCACGTG CTACGTAAAA GCCAGGGCGG CGTGTAACGC 240
CGCGGGGATT GTTGCTCCCA CCATGCTGGT GTGCGGTGAC 280
GACCTGGTCG TCATCTCAGA GAGTCAAGGG GCTGAGGAGG 320
ACGAGCAGAA CCTGAGAGTC 340

(2) INFORMATION FOR SEQ ID NO: 15

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5i10

(xi) SEQUENCE DESCRIPTION: SEQ ID NO: 15
CTCTACAGTC ACAGAGAGGG ACATCAGAAC CGAGGAGTCC 40
ATCTATCTGT CCTGCTCACT GCCTGAGGAG GCCCGAACTG 80
CTATACACTC ACTGACTGAG AGACTGTACG TAGGGGGGCC 120
CATGACAAAC AGCAAGGGGC AATCCTGCGG GTACAGGCGT 160
TGCCGCGCGA GCGGAGTGCT CACCACCAGC ATGGGCAACA 200
CGCTCACGTG CTACGTGAAA GCCAGAGCGG CGTGTAACGC 240

CGCGGGCATT GTTGCTCCCA CCATGTTGGT GTGCGGCGAC 280
GACCTGGTTG TCATCTCAGA GAGTCAGGGG GTCGAGGAAG 320
ATGAGCGGAA CCTGAGAGTC 340

(2) INFORMATION FOR SEQ ID NO: 16

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5arg6

(xi) SEQUENCE DESCRIPTION: SEQ ID NO: 16
CTCTACAGTC ACGGAGAGGG ACATCAGAAC CGAGGAGTCC 40
ATCTATCTGT CCTGTTCACT GCCTGAGGAG GCTCGAACTG 80
CCATACACTC ACTGACTGAG AGGCTGTACG TAGGGGGGCC 120
CATGACAAAC AGCAAAGGGC AATCCTGCGG GTACAGGCGT 160
TGCCGCGCGA GCGGAGTGCT CACCACCAGC ATGGGTAACA 200
CACTCACGTG CTACGTGAAA GCTAAAGCGG CATGTAACGC 240
CGCGGGCATT GTTGCCCCCA CCATGTTGGT GTGCGGCGAC 280
GACCTAGTCG TCATCTCAGA GAGTCAAGGG GTCGAGGAGG 320
ATGAGCGAAA CCTGAGAGCT 340

(2) INFORMATION FOR SEQ ID NO: 17

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5k2b

(xi) SEQUENCE DESCRIPTION: SEQ ID NO: 17
CTCAACCGTC ACGGAGAGGG ACATAAGAAC AGAAGAATCC 40
ATATATCAGG GTTGTTCCCT GCCTCAGGAG GCTAGAACTG 80
CTATCCACTC GCTCACTGAG AGACTCTACG TAGGAGGGCC 120
CATGACAAAC AGCAAGGGAC AATCCTGCGG TTACAGGCGT 160
TGCCGCGCCA GCGGGGTCTT CACCACCAGC ATGGGGAATA 200
CCATGACATG CTACATCAAA GCCCTTGCAG CGTGCAAAGC 240
TGCAGGGATC GTGGACCCTA TCATGCTGGT GTGTGGAGAC 280
GACCTGGTCG TCATCTCGGA GAGCGAAGGT AACGAGGAGG 320
ACGAGCGAAA CCTGAGAGCT 340

(2) INFORMATION FOR SEQ ID NO: 18

(i) SEQUENCE CHARACTERISTICS:

(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5sa283

(xi) SEQUENCE DESCRIPTION: SEQ ID NO: 18
CTCGACCGTT ACCGAACATG ACATAATGAC TGAAGAGTCT 40
ATTTACCAAT CATTGTACTT GCAGCCTGAG GCGCGTGTGG 80
CAATACGGTC ACTCACCCAA CGCCTGTACT GTGGAGGCCC 120
CATGTATAAC AGCAAGGGGC AACAATGTGG TTATCGTAGA 160
TGCCGCGCCA GCGGCGTCTT CACCACTAGT ATGGGCAACA 200
CCATGACGTG CTACATTAAG GCTTTAGCCT CCTGTAGAGC 240
CGCAAAGCTC CAGGACTGCA CGCTCCTGGT GTGTGGTGAT 320
GATAAAGCGA CCTGAGAGCC 340

(2) INFORMATION FOR SEQ ID NO: 19

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5sa156

(xi) SEQUENCE DESCRIPTION: SEQ ID NO: 19
CTCGACCGTT ACCGAACATG ACATAATGAC TGAAGAGTCC 40
ATTTACCAAT CATTGTACTT GCAGCCTGAG GCACGCGCGG 80
CAATACGGTC ACTCACCCAA CGCCTGTACT GTGGAGGCCC 120
CATGTATAAC AGCAAGGGGC AACAATGTGG TTACCGTAGA 160
TGCCGCGCCA GCGGCGTCTT CACCACCAGT ATGGGCAACA 200
CCATGACGTG CTACATCAAG GCTTCAGCCG CCTGTAGAGC 240
TGCAAAGCTC CAGGACTGCA CGCTCCTGGT GTGTGGTGTG 280
ACCTTGGTGG CCATTTGCGA GAGCCAAGGG ACGCACGAGG 320
ATGAAGCGTG CCTGAGAGTC 340

(2) INFORMATION FOR SEQ ID NO: 20

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:

(C) INDIVIDUAL ISOLATE: ns5i11

(xi) SEQUENCE DESCRIPTION: SEQ ID NO: 20
CTCTACTGTC ACTGAACAGG ACATCAGGGT GGAAGAGGAG 40
ATATACCAGT GCTGTAACCT TGAACCGGAG GCCAGGAAAG 80
TGATCTCCTC CCTCACGGAG CGGCTTTACT GCGGGGGCCC 120
TATGTTCAAC AGCAAGGGGG CCCAGTGTGG TTATCGCCGT 160
TGCCGTGCTA GTGGAGTCCT GCCTACCAGC TTCGGCAACA 200
CAATCACTTG TTACATCAAG GCTAGAGCGG CTTCGAAGGC 240
CGCAGGCCTC CGGAACCCGG ACTTTCTTGT CTGCGGAGAT 280
GATCTGGTCG TGGTGGCTGA GAGTGATGGC GTCGACGAGG 320
ATAGAGCAGC CCTGAGAGCC 340

(2) INFORMATION FOR SEQ ID NO: 21

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5i4

(xi) SEQUENCE DESCRIPTION: SEQ ID NO: 21

CTCGACTGTC ACTGAACAGG ACATCAGGGT GGAAGAGGAG 40
ATATACCAAT GCTGTAACCT TGAACCGGAG GCCAGGAAAG 80
TGATCTCCTC CCTCACGGAG CGGCTTTACT GCGGGGGCCC 120
TATGTTCAAT AGCAAGGGGG CCCAGTGTGG TTATCGCCGT 160
TGCCGTGCTA GTGGAGTTCT GCCTACCAGC TTCGGCAACA 200
CAATCACTTG TTACATCAAG GCTAGAGCGG CTGCGAAGGC 240
CGCAGGGCTC CGGACCCCGG ACTTTCTCGT CTGCGGAGAT 280
GATCTGGTTG TGGTGGCTGA GAGTGATGGC GTCGACGAGG 320
ATAGAACAGC CCTGCGAGCC 340

(2) INFORMATION FOR SEQ ID NO: 22

(i) SEQUENCE CHARACTERISTICS:
(A) LENGTH: 340 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: ns5gh8

(xi) SEQUENCE DESCRIPTION: SEQ ID NO: 22
CTCAACTGTC ACTGAACAGG ACATCAGGGT GGAAGAGGAG 40
ATATACCAAT GCTGTAACCT TGAACCGGAG GCCAGGAAAG 80
TGATCTCCTC CCTCACGGAA CGGCTTTACT GCGGGGGCCC 120

TATGTTCAAC AGCAAGGGGG CCCAGTGTGG TTATCGCCGT 160
TGCCGTGCCA GTGGAGTTCT GCCTACCAGC TTCGGCAACA 200
CAATCACTTG TTACATCAAA GCTAGAGCGG CTGCCGAAGC 240
CGCAGGCCTC CGGAACCCGG ACTTTCTTGT CTGCGGAGAT 280
GATCTGGTTG TGGTGGCTGA GAGTGATGGC GTCAATGAGG 320
ATAGAGCAGC CCTGGGAGCC 340

(2) INFORMATION FOR SEQ ID NO: 23

(i) SEQUENCE CHARACTERISTICS:
(A) LENGTH: 100 nucleotides
(B) TYPE: nucleic acid
(C) STRANDEDNESS: single
(D) TOPOLOGY: linear

(ii) MOLECULE TYPE: DNA

(vi) ORIGINAL SOURCE: (ATCC # 40394)
(C) INDIVIDUAL ISOLATE: hcv1

(xi) SEQUENCE DESCRIPTION: SEQ ID NO: 23
GACGGCGTTG GTAATGGCTC AGCTGCTCCG GATCCCACAA 40
GCCATCTTGG ACATGATCGC TGGTGCTCAC TGGGGAGTCC 80
TGGCGGGCAT AGCGTATTTC 100

(2) INFORMATION FOR SEQ ID NO: 24

(i) SEQUENCE CHARACTERISTICS:
(A) LENGTH: 100 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: US5

(xi) SEQUENCE DESCRIPTION: SEQ ID NO: 24
GACGGCGTTG GTGGTAGCTC AGGTACTCCG GATCCCACAA 40
GCCATCATGG ACATGATCGC TGGAGCCCAC TGGGGAGTCC 80
TGGCGGGCAT AGCGTATTTC 100

(2) INFORMATION FOR SEQ ID NO: 25

(i) SEQUENCE CHARACTERISTICS:
(A) LENGTH: 100 nucleotides
(B) TYPE: nucleic acid
(C) STRANDEDNESS: single
(D) TOPOLOGY: linear

(ii) MOLECULE TYPE: DNA

(vi) ORIGINAL SOURCE:

(C) INDIVIDUAL ISOLATE: AUS5

(xi) SEQUENCE DESCRIPTION: SEQ ID NO: 25
AACGGCGCTG GTAGTAGCTC AGCTGCTCAG GGTCCCGCAA 40
GCCATCGTGG ACATGATCGC TGGTGCCCAC TGGGGAGTCC 80
TAGCGGGCAT AGCGTATTTT 100

(2) INFORMATION FOR SEQ ID NO: 26

(i) SEQUENCE CHARACTERISTICS:
(A) LENGTH: 100 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: US4

(xi) SEQUENCE DESCRIPTION: SEQ ID NO: 26
GACAGCCCTA GTGGTATCGC AGTTACTCCG GATCCCACAA 40
GCCGTCATGG ATATGGTGGC GGGGGCCCAC TGGGGAGTCC 80
TGGCGGGCCT TGCCTACTAT 100

(2) INFORMATION FOR SEQ ID NO: 27

(i)    SEQUENCE CHARACTERISTICS:
       (A)   LENGTH: 100 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:
       (C)   INDIVIDUAL ISOLATE:  ARG2

(xi)   SEQUENCE DESCRIPTION: SEQ ID NO: 27
AGCAGCCCTA GTGGTGTCGC AGTTACTCCG GATCCCACAA    40
AGCATCGTGG ACATGGTGGC GGGGGCCCAC TGGGGAGTCC    80
TGGCGGGCCT TGCTTACTAT                         100

(2)  INFORMATION FOR SEQ ID NO: 28

(i)    SEQUENCE CHARACTERISTICS:
       (A)   LENGTH: 100 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:

(C) INDIVIDUAL ISOLATE: I15

(xi) SEQUENCE DESCRIPTION: SEQ ID NO: 28
GGCAGCCCTA GTGGTGTCGC AGTTACTCCG GATCCCGCAA 40
GCTGTCGTGG ACATGGTGGC GGGGGCCCAC TGGGGAATCC 80
TAGCGGGTCT TGCCTACTAT 100

(2) INFORMATION FOR SEQ ID NO: 29

(i) SEQUENCE CHARACTERISTICS:
(A) LENGTH: 100 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: GH8

(xi) SEQUENCE DESCRIPTION: SEQ ID NO: 29
TGTGGGTATG GTGGTGGCGC ACGTCCTGCG TTTGCCCCAG 40
ACCTTGTTCG ACATAATAGC CGGGGCCCAT TGGGGCATCT 80
TGGCGGGCTT GGCCTATTAC 100

(2) INFORMATION FOR SEQ ID NO: 30

(i)    SEQUENCE CHARACTERISTICS:
       (A)   LENGTH: 100 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:
       (C)   INDIVIDUAL ISOLATE:  I4

(xi)   SEQUENCE DESCRIPTION: SEQ ID NO: 30
TGTGGGTATG GTGGTAGCAC ACGTCCTGCG TCTGCCCCAG    40
ACCTTGTTCG ACATAATAGC CGGGGCCCAT TGGGGCATCT    80
TGGCAGGCCT AGCCTATTAC                         100

(2)  INFORMATION FOR SEQ ID NO: 31

(i)    SEQUENCE CHARACTERISTICS:
       (A)   LENGTH: 100 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:

(C)   INDIVIDUAL ISOLATE:  I11

(xi)   SEQUENCE DESCRIPTION: SEQ ID NO: 31
TGTGGGTATG GTGGTGGCGC AAGTCCTGCG TTTGCCCCAG     40
ACCTTGTTCG ACGTGCTAGC CGGGGCCCAT TGGGGCATCT     80
TGGCGGGCCT GGCCTATTAC                          100

(2)  INFORMATION FOR SEQ ID NO: 32

(i)    SEQUENCE CHARACTERISTICS:
(A)   LENGTH: 100 nucleotides
(B)   TYPE: nucleic acid
(C)   STRANDEDNESS:  single
(D)   TOPOLOGY: linear

(ii)   MOLECULE TYPE:  DNA

(vi)   ORIGINAL SOURCE:
(C)   INDIVIDUAL ISOLATE:  I10

(xi)   SEQUENCE DESCRIPTION: SEQ ID NO: 32
TACCACTATG CTCCTGGCAT ACTTGGTGCG CATCCCGGAG     40
GTCATCCTGG ACATTATCAC GGGAGGACAC TGGGGCGTGA     80
TGTTTGGCCT GGCTTATTTC                          100

(2)  INFORMATION FOR SEQ ID NO: 33

(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE: (ATCC # 40394)
(C) INDIVIDUAL ISOLATE: hcv1

(xi) SEQUENCE DESCRIPTION: SEQ ID NO: 33
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATCAACCC 120
GCTCAATGCC TGGAGATTTG GGCGTGCCCC CGCAAGACTG 160
CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA CC 252

(2) INFORMATION FOR SEQ ID NO: 34

(i) SEQUENCE CHARACTERISTICS:
(A) LENGTH: 252 nucleotides
(B) TYPE: nucleic acid
(C) STRANDEDNESS: single
(D) TOPOLOGY: linear

(ii)   MOLECULE TYPE:  DNA

(vi)   ORIGINAL SOURCE:
       (C)   INDIVIDUAL ISOLATE:  us5

(xi)   SEQUENCE DESCRIPTION: SEQ ID NO: 34
GTTAGTATGA GTGTCGTGCA GCCTCCAGGA CCCCCCCTCC    40
CGGGAGAGCC ATAGTGGTCT GCGGAACCGG TGAGTACACC    80
GGAATTGCCA GGACGACCGG GTCCTTTCTT GGATCAACCC   120
GCTCAATGCC TGGAGATTTG GGCGTGCCCC CGCAAGACTG   160
CTAGCCGAGT AGTGTTGGGT CGCGAAAGGC CTTGTGGTAC   200
TGCCTGATAG GGTGCTTGCG AGTGCCCCGG GAGGTCTCGT   240
AGACCGTGCA CC                                 252

(2)  INFORMATION FOR SEQ ID NO: 35

(i)    SEQUENCE CHARACTERISTICS:
       (A)   LENGTH: 252 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:
       (C)   INDIVIDUAL ISOLATE:  aus1

(xi) SEQUENCE DESCRIPTION: SEQ ID NO: 35
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATCAACCC 120
GCTCAATGCC TGGAGATTTG GGCACGCCCC CGCAAGATCA 160
CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA CC 252

(2) INFORMATION FOR SEQ ID NO: 36

(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: sp2

(xi) SEQUENCE DESCRIPTION: SEQ ID NO: 36
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATAAACCC 120
GCTCAATGCC TGGAGATTTG GGCGTGCCCC CGCGAGACTG 160

CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA CC 252

(2) INFORMATION FOR SEQ ID NO: 37

(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: gm2

(xi) SEQUENCE DESCRIPTION: SEQ ID NO: 37
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATCAACCC 120
GCTCAATGCC TGGAGATTTG GGCGTGCCCC CGCAAGACTG 160
CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA CC 252

(2) INFORMATION FOR SEQ ID NO: 38

(i)    SEQUENCE CHARACTERISTICS:
        (A)    LENGTH: 252 nucleotides
        (B)    TYPE: nucleic acid
        (C)    STRANDEDNESS:  single
        (D)    TOPOLOGY: linear

(ii)    MOLECULE TYPE:  DNA

(vi)    ORIGINAL SOURCE:
        (C)    INDIVIDUAL ISOLATE:  i21

(xi)    SEQUENCE DESCRIPTION: SEQ ID NO: 38
GTTAGTATGA GTGTCGTGCA GCCTCCAGGA CCCCCCCTCC    40
CGGGAGAGCC ATAGTGGTCT GCGGAACCGG TGAGTACACC    80
GGAATTGCCA GGACGACCGG GTCCTTTCTT GGATAAACCC   120
GCTCAATGCC TGGAGATTTG GGCGTGCCCC CGCAAGACTG   160
CTAGCCGAGT AGTGTTGGGT CGCGAAAGGC CTTGTGGTAC   200
TGCCTGATAG GGTGCTTGCG AGTGCCCCGG GAGGTCTCGT   240
AGACCGTGCA CC                                 252

(2)  INFORMATION FOR SEQ ID NO: 39

(i)    SEQUENCE CHARACTERISTICS:
        (A)    LENGTH: 252 nucleotides
        (B)    TYPE: nucleic acid
        (C)    STRANDEDNESS:  single
        (D)    TOPOLOGY: linear

(ii)   MOLECULE TYPE:  DNA

(vi)   ORIGINAL SOURCE:
       (C)   INDIVIDUAL ISOLATE:  us4

(xi)   SEQUENCE DESCRIPTION: SEQ ID NO: 39
GTTAGTATGA GTGTCGTGCA GCCTCCAGGA CCCCCCCTCC    40
CGGGAGAGCC ATAGTGGTCT GCGGAACCGG TGAGTACACC    80
GGAATTGCCA GGACGACCGG GTCCTTTCTT GGATCAACCC   120
GCTCAATGCC TGGAGATTTG GGCGTGCCCC CGCGAGACTG   160
CTAGCCGAGT AGTGTTGGGT CGCGAAAGGC CTTGTGGTAC   200
TGCCTGATAG GGTGCTTGCG AGTGCCCCGG GAGGTCTCGT   240
AGACCGTGCA CC                                 252

(2)   INFORMATION FOR SEQ ID NO: 40

(i)    SEQUENCE CHARACTERISTICS:
       (A)   LENGTH: 252 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:
       (C)   INDIVIDUAL ISOLATE:  jh1

(xi) SEQUENCE DESCRIPTION: SEQ ID NO: 40
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATCAACCC 120
GCTCAATGCC TGGAGATTTG GGCGTGCCCC CGCGAGACTG 160
CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA TC 252

(2) INFORMATION FOR SEQ ID NO: 41

(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: nac5

(xi) SEQUENCE DESCRIPTION: SEQ ID NO: 41
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATCAACCC 120
GCTCAATGCC TGGAGATTTG GGCGTGCCCC CGCGAGACTG 160

CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA CC 252

(2) INFORMATION FOR SEQ ID NO: 42
(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: arg2

(xi) SEQUENCE DESCRIPTION: SEQ ID NO: 42
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATCAACCC 120
GCTCAATGCC TGGAGATTTG GGCGTGCCCC CGCGAGACTG 160
CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA CC 252

(2) INFORMATION FOR SEQ ID NO: 43

(i)    SEQUENCE CHARACTERISTICS:
       (A)    LENGTH: 252 nucleotides
       (B)    TYPE: nucleic acid
       (C)    STRANDEDNESS:  single
       (D)    TOPOLOGY: linear

(ii)    MOLECULE TYPE:  DNA

(vi)    ORIGINAL SOURCE:
       (C)    INDIVIDUAL ISOLATE:  sp1

(xi)    SEQUENCE DESCRIPTION: SEQ ID NO: 43
GTTAGTATGA GTGTCGTGCA GCCTCCAGGA CCCCCCCTCC    40
CGGGAGAGCC ATAGTGGTCT GCGGAACCGG TGAGTACACC    80
GGAATTGCCA GGACGACCGG GTCCTTTCTT GGATCAACCC   120
GCTCAATGCC TGGAGATTTG GGCGTGCCCC CGCGAGACTG   160
CTAGCCGAGT AGTGTTGGGT CGCGAAAGGC CTTGTGGTAC   200
TGCCTGATAG GGTGCTTGCG AGTGCCCCGG GAGGTCTCGT   240
AGACCGTGCA CC                                 252

(2)  INFORMATION FOR SEQ ID NO: 44

(i)    SEQUENCE CHARACTERISTICS:
       (A)    LENGTH: 252 nucleotides
       (B)    TYPE: nucleic acid
       (C)    STRANDEDNESS:  single
       (D)    TOPOLOGY: linear

(ii)   MOLECULE TYPE:  DNA

(vi)   ORIGINAL SOURCE:
       (C)   INDIVIDUAL ISOLATE:  gh1

(xi)   SEQUENCE DESCRIPTION: SEQ ID NO: 44
GTTAGTATGA GTGTCGTGCA GCCTCCAGGA CCCCCCCTCC    40
CGGGAGAGCC ATAGTGGTCT GCGGAACCGG TGAGTACACC    80
GGAATTGCCA GGACGACCGG GTCCTTTCTT GGATCAACCC   120
GCTCAATGCC TGGAGATTTG GGCGTGCCCC CGCGAGACTG   160
CTAGCCGAGT AGTGTTGGGT CGCGAAAGGC CTTGTGGTAC   200
TGCCTGATAG GGTGCTTGCG AGTGCCCCGG GAGGTCTCGT   240
AGACCGTGCA CC                                 252

(2)   INFORMATION FOR SEQ ID NO: 45

(i)    SEQUENCE CHARACTERISTICS:
       (A)   LENGTH: 252 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:
       (C)   INDIVIDUAL ISOLATE:  i15

(xi) SEQUENCE DESCRIPTION: SEQ ID NO: 45
GTTAGTATGA GTGTCGTGCA GCCTCCAGGA CCCCCCCTCC 40
CGGGAGAGCC ATAGTGGTCT GCGGAACCGG TGAGTACACC 80
GGAATTGCCA GGACGACCGG GTCCTTTCTT GGATCAACCC 120
GCTCAATGCC TGGAGATTTG GGCGTGCCCC CGCGAGACTG 160
CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA CC 252

(2) INFORMATION FOR SEQ ID NO: 46

(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: i10

(xi) SEQUENCE DESCRIPTION: SEQ ID NO: 46
GCTAGTATCA GTGTCGTACA GCCTCCAGGC CCCCCCCTCC 40
CGGGAGAGCC ATAGTGGTCT GCGGAACCGG TGAGTACACC 80
GGAATTGCCG GGAAGACTGG GTCCTTTCTT GGATAAACCC 120
ACTCTATGCC CGGCCATTTG GGCGTGCCCC CGCAAGACTG 160
CTAGCCGAGT AGCGTTGGGT TGCGAAAGGC CTTGTGGTAC 200
TGCCTGATAG GGTGCTTGCG AGTGCCCCGG GAGGTCTCGT 240
AGACCGTGCA TC 252

(2) INFORMATION FOR SEQ ID NO: 47

(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: arg6

(xi) SEQUENCE DESCRIPTION: SEQ ID NO: 47
GTTAGTATGA GTCTCGTACA GCCTCCAGGC CCCCCCCTCC 40
CGGGAGAGCC ATAGTGGTCT GCGGAACCGG TGAGTACACC 80
GGAATTGCTG GGAAGACTGG GTCCTTTCTT GGATAAACCC 120
ACTCTATGCC CAGCCATTTG GGCGTGCCCC CGCAAGACTG 160

CTAGCCGAGT AGCGTTGGGT TGCGAAAGGC CTTGTGGTAC 200
TGCCTGATAG GGTGCTTGCG AGTGCCCCGG GAGGTCTCGT 240
AGACCGTGCA TC 252

(2) INFORMATION FOR SEQ ID NO: 48
(i) SEQUENCE CHARACTERISTICS:
(A) LENGTH: 252 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: s21

(xi) SEQUENCE DESCRIPTION: SEQ ID NO: 48

GTTAGTACGA GTGTCGTGCA GCCTCCAGGA CTCCCCCTCC 40
CGGGAGAGCC ATAGTGGTCT GCGGAACCGG TGAGTACACC 80
GGAATCGCTG GGGTGACCGG GTCCTTTCTT GGAGCAACCC 120
GCTCAATACC CAGAAATTTG GGCGTGCCCC CGCGAGATCA 160
CTAGCCGAGT AGTGTTGGGT CGCGAAAGGC CTTGTGGTAC 200
TGCCTGATAG GGTGCTTGCG AGTGCCCCGG GAGGTCTCGT 240
AGACCGTGCA AC 252

(2) INFORMATION FOR SEQ ID NO: 49

(i)    SEQUENCE CHARACTERISTICS:
       (A)   LENGTH: 252 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:
       (C)   INDIVIDUAL ISOLATE:  gj61329

(xi)   SEQUENCE DESCRIPTION: SEQ ID NO: 49

GTTAGTACGA GTGTCGTGCA GCCTCCAGGA CCCCCCCTCC    40
CGGGAGAGCC ATAGTGGTCT GCGGAACCGG TGAGTACACC    80
GGAATCGCTG GGGTGACCGG GTCCTTTCTT GGAGTAACCC   120
GCTCAATACC CAGAAATTTG GGCGTGCCCC CGCGAGATCA   160
CTAGCCGAGT AGTGTTGGGT CGCGAAAGGC CTTGTGGTAC   200
TGCCTGATAG GGTGCTTGCG AGTGCCCCGG GAGGTCTCGT   240
AGACCGTGCA AC                                 252

(2)  INFORMATION FOR SEQ ID NO: 50

(i)    SEQUENCE CHARACTERISTICS:
       (A)   LENGTH: 180 nucleotides

(B) TYPE: nucleic acid
(C) STRANDEDNESS: single
(D) TOPOLOGY: linear

(ii) MOLECULE TYPE: DNA
(vi) ORIGINAL SOURCE:
(C) INDIVIDUAL ISOLATE: sa3

(xi) SEQUENCE DESCRIPTION: SEQ ID NO: 50

GTTAGTATGA GTGTCGAACA GCCTCCAGGA CCCCCCCTCC 40
CGGGAGAGCC ATAGTGGTCT GCGGAACCGG TGAGTACACC 80
GGAATTGCCG GGATGACCGG GTCCTTTCTT GGATAAACCC 120
GCTCAATGCC CGGAGATTTG GGCGTGCCCC CGCGAGACTG 160
CTAGCCGAGT AGTGTTGGGT 180

(2) INFORMATION FOR SEQ ID NO: 51

(i) SEQUENCE CHARACTERISTICS:
(A) LENGTH: 180 nucleotides
(B) TYPE: nucleic acid
(C) STRANDEDNESS: single
(D) TOPOLOGY: linear

(ii) MOLECULE TYPE: DNA

(vi) ORIGINAL SOURCE:

(C)   INDIVIDUAL ISOLATE:  sa4

(xi)   SEQUENCE DESCRIPTION: SEQ ID NO: 51
GTTAGTATGA GTGTCGAACA GCCTCCAGGA CCCCCCCTCC    40
CGGGAGAGCC ATAGTGGTCT GCGGAACCGG TGAGTACACC    80

GGAATTGCCG GGATGACCGG GTCCTTTCTT GGATAAACCC   120
GCTCAATGCC CGGAGATTTG GGCGTGCCCC CGCGAGACTG   160
CTAGCCGAGT AGTGTTGGGT                         180

(2)  INFORMATION FOR SEQ ID NO: 52

(i)    SEQUENCE CHARACTERISTICS:
       (A)   LENGTH: 549 nucleotides
       (B)   TYPE: nucleic acid
       (C)   STRANDEDNESS:  single
       (D)   TOPOLOGY: linear

(ii)   MOLECULE TYPE:  DNA

(vi)   ORIGINAL SOURCE:  (ATCC # 40394)
       (C)   INDIVIDUAL ISOLATE:  hcv1

(xi) SEQUENCE DESCRIPTION: SEQ ID NO: 52
ATGAGCACGA ATCCTAAACC TCAAAAAAAA AACAAACGTA 40
ACACCAACCG TCGCCCACAG GACGTCAAGT TCCCGGGTGG 80
CGGTCAGATC GTTGGTGGAG TTTACTTGTT GCCGCGCAGG 120
GGCCCTAGAT TGGGTGTGCG CGCGACGAGA AAGACTTCCG 160
AGCGGTCGCA ACCTCGAGGT AGACGTCAGC CTATCCCCAA 200
GGCTCGTCGG CCCGAGGGCA GGACCTGGGC TCAGCCCGGG 240
TACCCTTGGC CCCTCTATGG CAATGAGGGC TGCGGGTGGG 280
CGGGATGGCT CCTGTCTCCC CGTGGCTCTC GGCCTAGCTG 320
GGGCCCCACA GACCCCCGGC GTAGGTCGCG CAATTTGGGT 360
AAGGTCATCG ATACCCTTAC GTGCGGCTTC GCCGACCTCA 400
TGGGGTACAT ACCGCTCGTC GGCGCCCCTC TTGGAGGCGC 440
TGCCAGGGCC CTGGCGCATG GCGTCCGGGT TCTGGAAGAC 480
GGCGTGAACT ATGCAACAGG GAACCTTCCT GGTTGCTCTT 520
TCTCTATCTT CCTTCTGGCC CTGCTCTCT 549

(2) INFORMATION FOR SEQ ID NO: 53

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:

(C) INDIVIDUAL ISOLATE: us5

(xi) SEQUENCE DESCRIPTION: SEQ ID NO: 53
ATGAGCACGA ATCCTAAACC TCAAAGAAAA ACCAAACGTA 40
ACACCAACCG TCGCCCACAG GACGTCAAGT TCCCGGGTGG 80
CGGTCAGATC GTTGGTGGAG TTTACTTGTT GCCGCGCAGG 120
GGCCCTAGAT TGGGTGTGCG CGCGACGAGG AAGACTTCCG 160
AGCGGTCGCA ACCTCGAGGT AGACGTCAGC CTATCCCCAA 200
GGCGCGTCGG CCCGAGGGCA GGACCTGGGC TCAGCCCGGG 240
TACCCTTGGC CCCTCTATGG CAATGAGGGT TGCGGGTGGG 280
CGGGATGGCT CCTGTCTCCC CGTGGCTCTC GGCCTAGTTG 320
GGGCCCCACA GACCCCCGGC GTAGGTCGCG CAATTTGGGT 360
AAGGTCATCG ATACCCTTAC GTGCGGCTTC GCCGACCACA 400
TGGGGTACAT ACCGCTCGTC GGCGCCCCTC TTGGAGGCGC 440
TGCCAGGGCT CTGGCGCATG GCGTCCGGGT TCTGGAAGAC 480
GGCGTGAACT ATGCAACAGG GAACCTTCCT GGTTGCTCTT 520
TCTCTATCTT CCTTCTGGCC CTGCTCTCT 549

(2) INFORMATION FOR SEQ ID NO: 54

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: aus1

(xi) SEQUENCE DESCRIPTION: SEQ ID NO: 54
ATGAGCACGA ATCCTAAACC TCAAAGAAAA ACCAAACGTA 40
ACACCAACCG TCGCCCACAG GACGTTAAGT TCCCGGGTGG 80
CGGTCAGATC GTTGGTGGAG TTTACTTGTT GCCGCGCAGG 120
GGCCCTAGAT TGGGTGTGCG CGCGACGAGG AAGACTTCCG 160
AGCGGTCGCA ACCTCGAGGT AGACGTCAGC CTATCCCTAA 200
GGCGCGTCGG CCCGAGGGCA GGACCTGGGC TCAGCCCGGG 240
TACCCCTGGC CCCTCTATGG TAATGAGGGT TGCGGATGGG 280
CGGGATGGCT CCTGTCCCCC CGTGGCTCTC GGCCTAGTTG 320
GGGCCCTACA GACCCCCGGC GTAGGTCGCG CAATTTGGGT 360
AAGGTCATCG ATACCCTCAC GTGCGGCTTC GCCGACCACA 400
TGGGGTACAT TCCGCTCGTT GGCGCCCCTC TTGGGGGCGC 440
TGCCAGGGCC CTGGCGCATG GCGTCCGGGT TCTGGAAGAC 480
GGCGTGAACT ATGCAACAGG GAATCTTCCT GGTTGCTCTT 520
TCTCTATCTT CCTTCTGGCC CTTCTCTCT 549

(2) INFORMATION FOR SEQ ID NO: 55

(i) SEQUENCE CHARACTERISTICS:
(A) LENGTH: 549 nucleotides
(B) TYPE: nucleic acid
(C) STRANDEDNESS: single

(D) TOPOLOGY: linear

(ii) MOLECULE TYPE: DNA

(vi) ORIGINAL SOURCE:
(C) INDIVIDUAL ISOLATE: sp2
(xi) SEQUENCE DESCRIPTION: SEQ ID NO: 55
ATGAGCACGA ATCCTAAACC TCAAAGAAAA ACCAAACGTA 40
ACACCAACCG TCGCCCACAG GACGTCAAGT TCCCGGGTGG 80
CGGTCAGATC GTTGGTGGAG TTTACTTGTT GCCGCGCAGG 120
GGCCCTAGAT TGGGTGTGCG CACGACGAGG AAGACTTCCG 160
AGCGGTCGCA ACCTCGAGGT AGACGTCAGC CCATCCCCAA 200
GGCTCGTCGA CCCGAGGGCA GGACCTGGGC TCAGCCCGGG 240
TACCCTTGGC CCCTCTATGG CAATGAGGGC TGCGGGTGGG 280
CGGGATGGCT CCTGTCTCCC CGTGGCTCTC GGCCTAGCTG 320
GGGCCCCACA GACCCCCGGC GTAGGTCGCG CAATTTGGGT 360
AAGGTCATCG ATACCCTTAC GTGCGGCTTC GCCGACCTCA 400
TGGGGTACAT ACCGCTCGTC GGCGCCCCTC TTGGAGGCGC 440
TGCCAGAGCC CTGGCGCATG GCGTCCGGGT TCTGGAAGAC 480
GGCGTGAACT ATGCAACAGG GAACCTTCCC GGTTGCTCTT 520
TCTCTATCTT CCTTCTGGCC CTGCTCTCT 549

(2) INFORMATION FOR SEQ ID NO: 56

(i) SEQUENCE CHARACTERISTICS:
(A) LENGTH: 549 nucleotides
(B) TYPE: nucleic acid

(C) STRANDEDNESS: single
(D) TOPOLOGY: linear

(ii) MOLECULE TYPE: DNA

(vi) ORIGINAL SOURCE:
(C) INDIVIDUAL ISOLATE: gm2

(xi) SEQUENCE DESCRIPTION: SEQ ID NO: 56
ATGAGCACGA ATCCTAAACC TCAAAGAAGA ACCAAACGTA 40
ACACCAACCG TCGCCCACAG GACGTCAAGT TCCCGGGTGG 80
CGGTCAGATC GTTGGTGGAG TTTACTTGTT GCCGCGCAGG 120
GGCCCTAGAT TGGGTGTGCG CGCGACGAGG AAGACTTCCG 160
AGCGGTCGCA ACCTCGAGGT AGACGTCAGC CTATCCCCAA 200
GGCACGTCGG CCCGAGGGTA GGACCTGGGC TCAGCCCGGG 240
TACCCTTGGC CCCTCTATGG CAATGAGGGT TGCGGGTGGG 280
CGGGATGGCT CCTGTCTCCC CGCGGCTCTC GGCCTAACTG 320
GGGCCCCACA GACCCCCGGC GTAGGTCGCG CAATTTGGGT 360
AAGGTCATCG ATACCCTTAC GTGCGGCTTC GCCGACCTCA 400
TGGGGTACAT ACCGCTCGTC GGCGCCCCTC TTGGAGGCGC 440
TGCCAGGGCC CTGGCGCATG GCGTCCGGGT TCTGGAAGAC 480
GGCGTGAACT ATGCAACAGG GAACCTTCCT GGTTGCTCTT 520
TCTCTATCTT CCTTCTGGCC CTGCTCTCT 549

(2) INFORMATION FOR SEQ ID NO: 57

(i) SEQUENCE CHARACTERISTICS:

(A) LENGTH: 549 nucleotides
(B) TYPE: nucleic acid
(C) STRANDEDNESS: single
(D) TOPOLOGY: linear

(ii) MOLECULE TYPE: DNA
(vi) ORIGINAL SOURCE:
(C) INDIVIDUAL ISOLATE: i21

(xi) SEQUENCE DESCRIPTION: SEQ ID NO: 57
ATGAGCACGA ATCCTAAACC TCAAAGAAAA ACCAAACGTA 40
ACACCAACCG TCGCCCACAG GACGTCAAGT TCCCGGGTGG 80
CGGTCAGATC GTTGGTGGAG TTTACTTGTT GCCGCGCAGG 120
GGCCCTAGAT TGGGTGTGCG CGCGACGAGG AAGACTTCCG 160
AGCGGTCGCA ACCTCGTGGT AGACGCCAGC CTATCCCCAA 200
GGCGCGTCGG CCCGAGGGCA GGACCTGGGC TCAGCCCGGG 240
TACCCTTGGC CCCTCTATGG CAATGAGGGT TGCGGGTGGG 280
CGGGATGGCT CCTGTCTCCC CGTGGCTCTC GGCCTAGCTG 320
GGGCCCCACA GACCCCCGGC GTAGGTCGCG CAATTTGGGT 360
AAGGTCATCG ATACCCTTAC GTGCGGCTTC GCCGACCTCA 400
TGGGGTACAT ACCGCTCGTC GGCGCCCCTC TTGGAGGCGC 440
TGCCAGGGCC CTGGCGCATG GCGTCCGGGT TCTGGAAGAC 480
GGCGTGAACT ATGCAACAGG GAACCTTCCT GGTTGCTCTT 520
TTTCTATTTT CCTTCTGGCC CTGCTCTCT 549

(2) INFORMATION FOR SEQ ID NO: 58

(i)    SEQUENCE CHARACTERISTICS:
       (A)   LENGTH: 549 nucleotides
       (B)   TYPE: nucleic acid
       (C)   STRANDEDNESS:  single
       (D)   TOPOLOGY: linear
(ii)   MOLECULE TYPE:  DNA

(vi)   ORIGINAL SOURCE:
       (C)   INDIVIDUAL ISOLATE:  us4

(xi)   SEQUENCE DESCRIPTION: SEQ ID NO: 58
ATGAGCACGA ATCCTAAACC TCAAAGAAAA ACCAAACGTA    40
ACACCAACCG CCGCCCACAG GACGTTAAGT TCCCGGGCGG    80
TGGCCAGGTC GTTGGTGGAG TTTACCTGTT GCCGCGCAGG   120
GGCCCCAGGT TGGGTGTGCG CGCGACTAGG AAGACTTCCG   160
AGCGGTCGCA ACCTCGTGGA AGGCGACAAC CTATCCCCAA   200
GGCTCGCCAG CCCGAGGGCA GGGCCTGGGC TCAGCCCGGG   240
TACCCTTGGC CCCTCTATGG CAATGAGGGT ATGGGGTGGG   280
CAGGATGGCT CCTGTCACCC CGTGGCTCTC GGCCTAGTTG   320
GGGCCCCACG GACCCCCGGC GTAGGTCGCG TAATTTGGGT   360
AAGGTCATCG ATACCCTCAC ATGCGGCTTC GCCGACCTCA   400
TGGGGTACAT TCCGCTCGTC GGCGCCCCCC TTAGGGGCGC   440
TGCCAGGGCC TTGGCGCATG GCGTCCGGGT TCTGGAGGAC   480
GGCGTGAACT ACGCAACAGG GAATCTGCCC GGTTGCTCCT   520
TTTCTATCTT CCTCTTGGCT CTGCTGTCC              549

(2) INFORMATION FOR SEQ ID NO: 59

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: jh1

(xi) SEQUENCE DESCRIPTION: SEQ ID NO: 59
ATGAGCACAA ATCCTAAACC TCAAAGAAAA ACCAAACGTA 40
ACACCAACCG CCGCCCACAG GACGTCAAGT TCCCGGGCGG 80
TGGTCAGATC GTTGGTGGAG TTTACCTGTT GCCGCGCAGG 120
GGCCCCAGGT TGGGTGTGCG CGCGACTAGG AAGACTTCCG 160
AGCGGTCGCA ACCTCGTGGA AGGCGACAAC CTATCCCCAA 200
GGCTCGCCAG CCCGAGGGCA GGGCCTGGGC TCAGCCCGGG 240
TACCCTTGGC CCCTCTATGG CAACGAGGGT ATGGGTGGGG 280
CAGGATGGCT CCTGTCACCC CGTGGCTCTC GGCCTAGTTG 320
GGGCCCCACG GACCCCCGGC GTAGGTCGCG TAATTTGGGT 360
AAGGTCATCG ATACCCTCAC ATGCGGCTTC GCCGACCTCA 400
TGGGGTACAT TCCGCTTGTC GGCGCCCCCC TAGGGGGCGC 440
TGCCAGGGCC CTGGCACATG GTGTCCGGGT TCTGGAGGAC 480
GGCGTGAACT ATGCAACAGG GAATTTGCCC GGTTGCTCTT 520

TCTCTATCTT CCTCTTGGCT CTGCTGTCC 549

(2) INFORMATION FOR SEQ ID NO: 60

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: nac5

(xi) SEQUENCE DESCRIPTION: SEQ ID NO: 60
ATGAGCACAA ATCCTAAACC CCAAAGAAAA ACCAAACGTA 40
ACACCAACCG TCGCCCACAG GACGTCAAGT TCCCGGGCGG 80
TGGTCAGATC GTTGGTGGAG TTTACCTGTT GCCGCGCAGG 120
GGCCCCAGGT TGGGTGTGCG CGCGACTAGG AAGACTTCCG 160
AGCGGTCGCA ACCTCGTGGA AGGCGACAAC CTATCCCCAA 200
GGCTCGCCGG CCCGAGGGCA GGTCCTGGGC TCAGCCCGGG 240
TACCCTTGGC CCCTCTATGG CAACGAGGGT ATGGGGTGGG 280
CAGGATGGCT CCTGTCACCC CGCGGCTCCC GGCCTAGTTG 320
GGGCCCCACG GACCCCCGGC GTAGGTCGCG TAATTTGGGT 360
AAGGTCATCG ATACCCTCAC ATGCGGCTTC GCCGACCTCA 400

TGGGGTACAT TCCGCTCGTC GGCGCCCCCC TAGGGGGCGC 440
TGCCAGGGCC CTGGCACATG GTGTCCGGGT TCTGGAGGAC 480
GGCGTGAACT ATGCAACAGG GAATTTGCCT GGTTGCTCTT 520
TCTCTATCTT CCTCTTGGCT CTGCTGTCC 549

(2) INFORMATION FOR SEQ ID NO: 61
(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: arg2

(xi) SEQUENCE DESCRIPTION: SEQ ID NO: 61
ATGAGCACGA ATCCTAAACC TCAAAGAAAA ACCAAACGTA 40
ACACCAACCG CCGCCCACAG GACGTCAAGT TCCCGGGCGG 80
TGGTCAGATC GTTGGTGGAG TTTACTTGTT GCCGCGCAGG 120
GGCCCCAGGT TGGGTGTGCG CGCGACTAGG AAGACTTCCG 160
AGCGGTCGCA ACCTCGTGGA AGGCGACAAC CTATCCCCAA 200
GGCTCGCCAG CCCGAGGGTA GGGCCTGGGC TCAGCCCGGG 240
TACCCTTGGC CCCTCTATGG CAATGAGGGT ATGGGGTGGG 280
CAGGGTGGCT CCTGTCCCCC CGCGGCTCCC GGCCTAGTTG 320

GGGCCCCACA GACCCCCGGC GTAGGTCGCG TAATTTGGGT 360
AAGGTCATCG ATACCCTCAC ATGCGGCTTC GCCGACCTCA 400
TGGGGTACAT TCCGCTCGTC GGCGCCCCCC TAGGGGGCGC 440
TGCCAGGGCC CTGGCGCATG GCGTCCGGGT TCTGGAGGAC 480
GGCGTGAACT ATGCAACAGG GAATCTGCCC GGTTGCTCTT 520
TCTCTATCTT CCTCTTGGCT TTGCTGTCC 549
(2) INFORMATION FOR SEQ ID NO: 62

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: sp1

(xi) SEQUENCE DESCRIPTION: SEQ ID NO: 62
ATGAGCACGA ATCCTAAACC TCAAAGAAAA ACCAAACGTA 40
ACACCAACCG CCGCCCACAG GACGTCAAGT TCCCGGGCGG 80
TGGTCAGATC GTTGGTGGAG TTTACCTGTT GCCGCGCAGG 120
GGCCCCAGGT TGGGTGTGCG CGCGACTAGG AAGACTTCCG 160
AGCGGTCGCA ACCTCGTGGA AGGCGACAAC CTATCCCCAA 200
GGCTCGCCGG CCCGAGGGCA GGGCCTGGGC TCAGCCCGGG 240
TATCCTTGGC CCCTCTATGG CAATGAGGGT CTGGGGTGGG 280

CAGGATGGCT CCTGTCACCC CGCGGCTCTC GGCCTAGCTG 320
GGGCCCTACC GACCCCCGGC GTAGGTCGCG CAACTTGGGT 360
AAGGTCATCG ATACCCTTAC GTGCGGCTTC GCCGACCTCA 400
TGGGGTACAT TCCGCTCGTC GGCGCCCCCC TTAGGGGCGC 440
TGCCAGGGCC CTGGCGCATG GCGTCCGGGT TCTGGAGGAC 480
GGCGTGAACT ATGCAACAGG GAATTTGCCC GGTTGCTCTT 520
TCTCTATCTT CCTCTTGGCT TTGCTGTCC 549

(2) INFORMATION FOR SEQ ID NO: 63

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: gh1

(xi) SEQUENCE DESCRIPTION: SEQ ID NO: 63
ATGAGCACGA ATCCTAAACC TCAAAGAAAA ACCAAACGTA 40
ACACCAACCG CCGCCCACAG GACGTCAAGT TCCCGGGCGG 80
TGGTCAGATC GTTGGTGGAG TTTACTTGTT GCCGCGCAGG 120
GGCCCCAGGT TGGGTGTGCG CGCGACTAGG AAGACTTCCG 160
AGCGGTCGCA ACCTCGTGGA AGGCGACAAC CTATCCCCAA 200

GGCTCGCCGG CCCGAGGGCA GGGCCTGGGC TCAGCCCGGG 240
TACCCTTGGC CCCTCTATGG CAATGAGGGT ATGGGGTGGG 280
CAGGATGGCT CCTGTCACCC CGTGGTTCTC GGCCTAGTTG 320
GGGCCCCACG GACCCCCGGC GTAGGTCGCG CAATTTGGGT 360
AAGATCATCG ATACCCTCAC GTGCGGCTTC GCCGACCTCA 400
TGGGGTACAT TCCGCTCGTC GGCGCCCCCC TAGGGGGCGC 440
TGCCAGGGCC CTGGCGCATG GCGTCCGGGT TCTGGAGGAC 480
GGCGTGAACT ATGCAACAGG GAATCTGCCC GGTTGCTCCT 520
TTTCTATCTT CCTTCTGGCT TTGCTGTCC 549

(2) INFORMATION FOR SEQ ID NO: 64

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: i15

(xi) SEQUENCE DESCRIPTION: SEQ ID NO: 64
ATGAGCACGA ATCCTAAACC TCAAAGAAAA ACCAAACGTA 40
ACACCAACCG CCGCCCACAG GACGTCAAGT TCCCGGGCGG 80
TGGTCAGATC GTTGGTGGAG TTTACCTGTT GCCGCGCAGG 120

GGCCCCAGGT TGGGTGTGCG CGCGACTAGG AAGACTTCCG 160
AGCGGTCGCA ACCTCGTGGA AGGCGACAAC CTATCCCCAA 200
GGCTCGCCAG CCCGAGGGCA GGGCCTGGGC TCAGCCCGGG 240
TACCCCTGGC CCCTCTATGG CAATGAGGGT ATGGGGTGGG 280
CAGGATGGCT CCTGTCACCC CGCGGCTCCC GGCCTAGTTG 320
GGGCCCCAAA GACCCCCGGC GTAGGTCGCG TAATTTGGGT 360
AAGGTCATCG ATACCCTCAC ATGCGGCTTC GCCGACCTCA 400
TGGGGTACAT TCCGCTCGTC GGCGCCCCCT TAGGGGGCGC 440
TGCCAGGGCC CTGGCGCATG GCGTCCGGGT TCTGGAGGAC 480
GGCGTGAACT ATGCAACAGG GAATCTACCC GGTTGCTCTT 520
TCTCTATCTT CCTCTTGGCT TTGCTGTCC 549

(2) INFORMATION FOR SEQ ID NO: 65

(i) SEQUENCE CHARACTERISTICS:
(A) LENGTH: 549 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: i10

(xi) SEQUENCE DESCRIPTION: SEQ ID NO: 65
ATGAGCACAA ATCCTAAACC TCAAAGAAAA ACCAAAAGAA 40

ACACTAACCG CCGCCCACAG GACGTCAAGT TCCCGGGCGG 80
TGGCCAGATC GTTGGCGGAG TATACTTGCT GCCGCGCAGG 120
GGCCCGAGAT TGGGTGTGCG CGCGACGAGG AAAACTTCCG 160
AACGATCCCA GCCACGCGGA AGGCGTCAGC CCATCCCTAA 200
AGATCGTCGC ACCGCTGGCA AGTCCTGGGG AAGGCCAGGA 240
TATCCTTGGC CCCTGTATGG GAATGAGGGT CTCGGCTGGG 280
CAGGGTGGCT CCTGTCCCCC CGTGGCTCTC GCCCTTCATG 320
GGGCCCCACT GACCCCCGGC ATAGATCGCG CAACTTGGGT 360
AAGGTCATCG ATACCCTAAC GTGCGGTTTT GCCGACCTCA 400
TGGGGTACAT TCCCGTCATC GGCGCCCCCG TTGGAGGCGT 440
TGCCAGAGCT CTCGCCCACG GAGTGAGGGT TCTGGAGGAT 480
GGGGTAAATT ATGCAACAGG GAATTTGCCC GGTTGCTCTT 520
TCTCTATCTT TCTCTTAGCC CTCTTGTCT 549

(2) INFORMATION FOR SEQ ID NO: 66

(i) SEQUENCE CHARACTERISTICS:
(A) LENGTH: 510 nucleotides
(B) TYPE: nucleic acid
(C) STRANDEDNESS: single
(D) TOPOLOGY: linear

(ii) MOLECULE TYPE: DNA

(vi) ORIGINAL SOURCE:
(C) INDIVIDUAL ISOLATE: arg6

(xi) SEQUENCE DESCRIPTION: SEQ ID NO: 66
ATGAGCACAA ATCCTCAACC TCAAAGAAAA ACCAAAAGAA 40
ACACTAACCG CCGCCCACAG GACGTCAAGT TCCCGGGCGG 80
TGGTCAGATC GTTGGCGGAG TATACTTGTT GCCGCGCAGG 120
GGCCCCAGGT TGGGTGTGCG CGCGACGAGG AAAACTTCCG 160
AACGGTCCCA GCCACGTGGG AGGCGCCAGC CCATCCCCAA 200
AGATCGGCGC ACCACTGGCA AGTCCTGGGG GAAGCCAGGA 240
TACCCTTGGC CCCTGTATGG GAATGAGGGT CTCGGCTGGG 280
CAGGGTGGCT CCTGTCCCCC CGCGGTTCTC GCCCTTCATG 320
GGGCCCCACT GACCCCCGGC ATAGATCACG CAACTTGGGT 360
AAGGTCATCG ATACCCTAAC GTGTGGTTTT GCCGACCTCA 400
TGGGGTACAT TCCCGTCGGT GGTGCCCCCG TTGGTGGTGT 440
CGCCAGAGCC CTTGCCCATG GGGTGAGGGT TCTGGAAGAC 480
GGGATAAATT ATGCAACAGG GAATCTGCCC 510

(2) INFORMATION FOR SEQ ID NO: 67

(i) SEQUENCE CHARACTERISTICS:
(A) LENGTH: 29 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 67
CAAACGTAAC ACCAACCGRC GCCCACAGG 29

(2) INFORMATION FOR SEQ ID NO: 68

(i) SEQUENCE CHARACTERISTICS:
(A) LENGTH: 24 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 68
ACAGAYCCGC AKAGRTCCCC CACG 24

(2) INFORMATION FOR SEQ ID NO: 69

(i) SEQUENCE CHARACTERISTICS:
(A) LENGTH: 30 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 69
CGAACCTCGA GGTAGACGTC AGCCTATCCC 30

(2) INFORMATION FOR SEQ ID NO: 70

(i) SEQUENCE CHARACTERISTICS:
(A) LENGTH: 30 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 70
GCAACCTCGT GGAAGGCGAC AACCTATCCC 30

(2) INFORMATION FOR SEQ ID NO: 71

(i) SEQUENCE CHARACTERISTICS:
(A) LENGTH: 30 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 71
GTCACCAATG ATTGCCCTAA CTCGAGTATT 30

(2) INFORMATION FOR SEQ ID NO: 72

(i) SEQUENCE CHARACTERISTICS:
(A) LENGTH: 26 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 72
GTCACGAACG ACTGCTCCAA CTCAAG 26

(2) INFORMATION FOR SEQ ID NO: 73

(i) SEQUENCE CHARACTERISTICS:
(A) LENGTH: 28 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 73
TGGACATGAT CGCTGGWGCY CACTGGGG 28

(2) INFORMATION FOR SEQ ID NO: 74

(i) SEQUENCE CHARACTERISTICS:
(A) LENGTH: 28 nucleotides
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO: 74
TGGAYATGGT GGYGGGGGCY CACTGGGG 28

(2) INFORMATION FOR SEQ ID NO: 75

(i) SEQUENCE CHARACTERISTICS:
(A) LENGTH: 20 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 75
ATGATGAACT GGTCVCCYAC 20

(2) INFORMATION FOR SEQ ID NO: 76

(i) SEQUENCE CHARACTERISTICS:

(A) LENGTH: 26 nucleotides
(B) TYPE: nucleic acid
(C) STRANDEDNESS: single
(D) TOPOLOGY: linear

(ii) MOLECULE TYPE: DNA
(xi) SEQUENCE DESCRIPTION: SEQ ID NO: 76
ACCTTVGCCC AGTTSCCCRC CATGGA 26

(2) INFORMATION FOR SEQ ID NO: 77

(i) SEQUENCE CHARACTERISTICS:
(A) LENGTH: 22 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 77
AACCCACTCT ATGYCCGGYC AT 22

(2) INFORMATION FOR SEQ ID NO: 78

(i) SEQUENCE CHARACTERISTICS:
(A) LENGTH: 18 nucleotides
(B) TYPE: nucleic acid

(C) STRANDEDNESS: single
(D) TOPOLOGY: linear

(ii) MOLECULE TYPE: DNA

(xi) SEQUENCE DESCRIPTION: SEQ ID NO: 78
GAATCGCTGG GGTGACCG 18

(2) INFORMATION FOR SEQ ID NO: 79

(i) SEQUENCE CHARACTERISTICS:
(A) LENGTH: 28 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 75
CCATGAATCA CTCCCCTGTG AGGAACTA 28

(2) INFORMATION FOR SEQ ID NO: 80

(i) SEQUENCE CHARACTERISTICS:
(A) LENGTH: 18 nucleotides
(B) TYPE: nucleic acid
(C) STRANDEDNESS: single

(D) TOPOLOGY: linear

(ii) MOLECULE TYPE: DNA

(xi) SEQUENCE DESCRIPTION: SEQ ID NO: 80
TTGCGGGGC ACGCCCAA 18
(2) INFORMATION FOR SEQ ID NO: 81

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 81
YGAAGCGGGC ACAGTCARRC AAGARAGCAG GGC 33

(2) INFORMATION FOR SEQ ID NO: 82

(i) SEQUENCE CHARACTERISTICS:
(A) LENGTH: 33 nucleotides
(B) TYPE: nucleic acid
(C) STRANDEDNESS: single

(D) TOPOLOGY: linear

(ii) MOLECULE TYPE: DNA

(xi) SEQUENCE DESCRIPTION: SEQ ID NO: 82
RTARAGCCCY GWGGAGTTGC GCACTTGGTR GGC 33
(2) INFORMATION FOR SEQ ID NO: 83

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 83
RATACTCGAG TTAGGGCAAT CATTGGTGAC RTG 33

(2) INFORMATION FOR SEQ ID NO: 84

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 84
AGYRTGCAGG ATGGYATCRK BCGYCTCGTA CAC 33

(2) INFORMATION FOR SEQ ID NO: 85
(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 85
GTTRCCCTCR CGAACGCAAG GGACRCACCC CGG 33

(2) INFORMATION FOR SEQ ID NO: 86

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 86
CGTRGGGGTY AYCGCCACCC AACACCTCGA GRC 33

(2) INFORMATION FOR SEQ ID NO: 87

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 87
CGTYGYGGGG AGTTTGCCRT CCCTGGTGGC YAC 33

(2) INFORMATION FOR SEQ ID NO: 88

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 88

CCCGACAAGC AGATCGATGT GACGTCGAAG CTG 33

(2) INFORMATION FOR SEQ ID NO: 89

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 89
CCCCACGTAG ARGGCCGARC AGAGRGTGGC GCY 33

(2) INFORMATION FOR SEQ ID NO: 90

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 90
YTGRCCGACA AGAAAGACAG ACCCGCAYAR GTC 33

(2) INFORMATION FOR SEQ ID NO: 91

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 91
CGTCCAGTGG YGCCTGGGAG AGAAGGTGAA CAG 33

(2) INFORMATION FOR SEQ ID NO: 92

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 92
GCCGGGATAG ATRGARCAAT TGCARYCTTG CGT 33

(2) INFORMATION FOR SEQ ID NO: 93

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 93
CATATCCCAT GCCATGCGGT GACCCGTTAY ATG 33

(2) INFORMATION FOR SEQ ID NO: 94

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 94
YACCAAYGCC GTCGTAGGGG ACCARTTCAT CAT 33

(2) INFORMATION FOR SEQ ID NO: 95

(i) SEQUENCE CHARACTERISTICS:
(A) LENGTH: 33 nucleotides
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO: 95
GATGGCTTGT GGGATCCGGA GYASCTGAGC YAY 33

(2) INFORMATION FOR SEQ ID NO: 96

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 96
GACTCCCCAG TGRGCWCCAG CGATCATRTC CAW 33

(2) INFORMATION FOR SEQ ID NO: 97

(i) SEQUENCE CHARACTERISTICS:

(A) LENGTH: 33 nucleotides
(B) TYPE: nucleic acid
(C) STRANDEDNESS: single
(D) TOPOLOGY: linear

(ii) MOLECULE TYPE: DNA
(xi) SEQUENCE DESCRIPTION: SEQ ID NO: 97
CCCCACCATG GAGAAATACG CTATGCCCGC YAG 33

(2) INFORMATION FOR SEQ ID NO: 98

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 98
TAGYAGCAGY ACTACYARGA CCTTCGCCCA GTT 33

(2) INFORMATION FOR SEQ ID NO: 99

(i) SEQUENCE CHARACTERISTICS:
(A) LENGTH: 33 nucleotides
(B) TYPE: nucleic acid

(C) STRANDEDNESS: single
(D) TOPOLOGY: linear

(ii) MOLECULE TYPE: DNA

(xi) SEQUENCE DESCRIPTION: SEQ ID NO: 99
GSTGACGTGR GTKTCYGCGT CRACGCCGGC RAA 33

(2) INFORMATION FOR SEQ ID NO: 100

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 100
GGAAGYTGGG ATGGTYARRC ARGASAGCAR AGC 33

(2) INFORMATION FOR SEQ ID NO: 101

(i) SEQUENCE CHARACTERISTICS:
(A) LENGTH: 33 nucleotides
(B) TYPE: nucleic acid
(C) STRANDEDNESS: single

(D) TOPOLOGY: linear

(ii) MOLECULE TYPE: DNA

(xi) SEQUENCE DESCRIPTION: SEQ ID NO: 101
GTAYAYYCCG GACRCGTTGC GCACTTCRTA AGC 33
(2) INFORMATION FOR SEQ ID NO: 102

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 102
AATRCTTGMG TTGGAGCART CGTTYGTGAC ATG 33

(2) INFORMATION FOR SEQ ID NO: 103

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 103
RGYRTGCATG ATCAYGTCCG YYGCCTCATA CAC 33

(2) INFORMATION FOR SEQ ID NO: 104
(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 104
RTTGTYYTCC CGRACGCARG GCACGCACCC RGG 33

(2) INFORMATION FOR SEQ ID NO: 105

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 105
CGTGGGRGTS AGCGCYACCC AGCARCGGGA GSW 33

(2) INFORMATION FOR SEQ ID NO: 106

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 106
YGTRGTGGGG AYGCTGKHRT TCCTGGCCGC VAR 33

(2) INFORMATION FOR SEQ ID NO: 107

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 107

CCCRACGAGC AARTCGACRT GRCGTCGTAW TGT 33

(2) INFORMATION FOR SEQ ID NO: 108

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 108
YCCCACGTAC ATAGCSGAMS AGARRGYAGC CGY 33

(2) INFORMATION FOR SEQ ID NO: 109

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 109
CTGGGAGAYR AGRAAAACAG ATCCGCARAG RTC 33

(2) INFORMATION FOR SEQ ID NO: 110

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 110
YGTCTCRTGC CGGCCAGSBG AGAAGGTGAA YAG 33

(2) INFORMATION FOR SEQ ID NO: 111

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 111
GCCGGGATAG AKKGAGCART TGCAKTCCTG YAC 33

(2) INFORMATION FOR SEQ ID NO: 112

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 112
CATATCCCAA GCCATRCGRT GGCCTGAYAC CTG 33

(2) INFORMATION FOR SEQ ID NO: 113

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 113
CACTARGGCT GYYGTRGGYG ACCAGTTCAT CAT 33

(2) INFORMATION FOR SEQ ID NO: 114

(i)    SEQUENCE CHARACTERISTICS:
       (A)   LENGTH: 33 nucleotides
       (B)   TYPE: nucleic acid
       (C)   STRANDEDNESS:  single
       (D)   TOPOLOGY: linear
(ii)   MOLECULE TYPE:  DNA

(xi)   SEQUENCE DESCRIPTION: SEQ ID NO: 114
GACRGCTTGT GGGATCCGGA GTAACTGCGA YAC                33

(2)  INFORMATION FOR SEQ ID NO: 115

(i)    SEQUENCE CHARACTERISTICS:
       (A)   LENGTH: 33 nucleotides
       (B)   TYPE: nucleic acid
       (C)   STRANDEDNESS:  single
       (D)   TOPOLOGY: linear

(ii)   MOLECULE TYPE:  DNA

(xi)   SEQUENCE DESCRIPTION: SEQ ID NO: 115
GACTCCCCAG TGRGCCCCCG CCACCATRTC CAT                33

(2)  INFORMATION FOR SEQ ID NO: 116

(i)    SEQUENCE CHARACTERISTICS:

(A) LENGTH: 33 nucleotides
(B) TYPE: nucleic acid
(C) STRANDEDNESS: single
(D) TOPOLOGY: linear

(ii) MOLECULE TYPE: DNA
(xi) SEQUENCE DESCRIPTION: SEQ ID NO: 116
SCCCACCATG GAWWAGTAGG CAAGGCCCGC YAG 33

(2) INFORMATION FOR SEQ ID NO: 117

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 117
GAGTAGCATC ACAATCAADA CCTTAGCCCA GTT 33

(2) INFORMATION FOR SEQ ID NO: 118

(i) SEQUENCE CHARACTERISTICS:
(A) LENGTH: 33 nucleotides
(B) TYPE: nucleic acid

(C) STRANDEDNESS: single
(D) TOPOLOGY: linear

(ii) MOLECULE TYPE: DNA

(xi) SEQUENCE DESCRIPTION: SEQ ID NO: 118
YGWCRYGYRG GTRTKCCCGT CAACGCCGGC AAA 33

(2) INFORMATION FOR SEQ ID NO: 119

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 119
TCCTCACAGG GGAGTGATTC ATGGTGGAGT GTC 33

(2) INFORMATION FOR SEQ ID NO: 120

(i) SEQUENCE CHARACTERISTICS:
(A) LENGTH: 33 nucleotides
(B) TYPE: nucleic acid
(C) STRANDEDNESS: single

(D) TOPOLOGY: linear

(ii) MOLECULE TYPE: DNA

(xi) SEQUENCE DESCRIPTION: SEQ ID NO: 120
ATGGCTAGAC GCTTTCTGCG TGAAGACAGT AGT 33
(2) INFORMATION FOR SEQ ID NO: 121

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 121
GCCTGGAGGC TGCACGRCAC TCATACTAAC GCC 33

(2) INFORMATION FOR SEQ ID NO: 122

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 122
CGCAGACCAC TATGGCTCTY CCGGGAGGGG GGG 33

(2) INFORMATION FOR SEQ ID NO: 123
(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 123
TCRTCCYGGC AATTCCGGTG TACTCACCGG TTC 33

(2) INFORMATION FOR SEQ ID NO: 124

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 124
GCATIGAGCG GGTTDATCCA AGAAAGGACC CGG 33

(2) INFORMATION FOR SEQ ID NO: 125

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 125
AGCAGTCTYG CGGGGGCACG CCCAARTCTC CAG 33

(2) INFORMATION FOR SEQ ID NO: 126

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 126

ACAAGGCCTT TCGCGACCCA ACACTACTCG GCT 33

(2) INFORMATION FOR SEQ ID NO: 127

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 127
GGGGCACTCG CAAGCACCCT ATCAGGCAGT ACC 33

(2) INFORMATION FOR SEQ ID NO: 128

(i) SEQUENCE CHARACTERISTICS:
(A) LENGTH: 33 nucleotides
(B) TYPE: nucleic acid
(C) STRANDEDNESS: single
(D) TOPOLOGY: linear

(ii)   MOLECULE TYPE:  DNA

(xi)   SEQUENCE DESCRIPTION: SEQ ID NO: 128
YGTGCTCATG RTGCACGGTC TACGAGACCT CCC                33

(2)   INFORMATION FOR SEQ ID NO: 129

(i)    SEQUENCE CHARACTERISTICS:
(A)    LENGTH: 33 nucleotides
(B)    TYPE: nucleic acid
(C)    STRANDEDNESS:  single
(D)    TOPOLOGY: linear

(ii)   MOLECULE TYPE:  DNA

(xi)   SEQUENCE DESCRIPTION: SEQ ID NO: 129
GTTACGTTTG KTTYTTYTTT GRGGTTTRGG AWT                33

(2)   INFORMATION FOR SEQ ID NO: 130

(i)    SEQUENCE CHARACTERISTICS:
(A)    LENGTH: 33 nucleotides
(B)    TYPE: nucleic acid
(C)    STRANDEDNESS:  single
(D)    TOPOLOGY: linear

(ii) MOLECULE TYPE: DNA

(xi) SEQUENCE DESCRIPTION: SEQ ID NO: 130
CGGGAACTTR ACGTCCTGTG GGCGRCGGTT GGT 33

(2) INFORMATION FOR SEQ ID NO: 131

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 131
CARGTAAACT CCACCRACGA TCTGRCCRCC RCC 33

(2) INFORMATION FOR SEQ ID NO: 132

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 132
RCGCACACCC AAYCTRGGGC CCCTGCGCGG CAA 33

(2) INFORMATION FOR SEQ ID NO: 133

(i) SEQUENCE CHARACTERISTICS:
(A) LENGTH: 33 nucleotides
(B) TYPE: nucleic acid
(C) STRANDEDNESS: single
(D) TOPOLOGY: linear
(ii) MOLECULE TYPE: DNA

(xi) SEQUENCE DESCRIPTION: SEQ ID NO: 133
AGGTTGCGAC CGCTCGGAAG TCTTYCTRGT CGC 33

(2) INFORMATION FOR SEQ ID NO: 134

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 134

RCGHRCCTTG GGGATAGGCT GACGTCWACC TCG 33

(2) INFORMATION FOR SEQ ID NO: 135

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA
(xi) SEQUENCE DESCRIPTION: SEQ ID NO: 135
RCGHRCCTTG GGGATAGGTT GTCGCCWTCC ACG 33

(2) INFORMATION FOR SEQ ID NO: 136

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 136
YCCRGGCTGR GCCCAGRYCC TRCCCTCGGR YYG 33

(2) INFORMATION FOR SEQ ID NO: 137

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 137
BSHRCCCTCR TTRCCRTAGA GGGGCCADGG RTA 33

(2) INFORMATION FOR SEQ ID NO: 138

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 138
GCCRCGGGGW GACAGGAGCC ATCCYGCCCA CCC 33

(2) INFORMATION FOR SEQ ID NO: 139

(i)    SEQUENCE CHARACTERISTICS:
       (A)   LENGTH: 33 nucleotides
       (B)   TYPE: nucleic acid
       (C)   STRANDEDNESS:  single
       (D)   TOPOLOGY: linear

(ii)   MOLECULE TYPE:  DNA

(xi)   SEQUENCE DESCRIPTION: SEQ ID NO: 139
CCGGGGGTCY GTGGGGCCCC AYCTAGGCCG RGA                33
(2)  INFORMATION FOR SEQ ID NO: 140

(i)    SEQUENCE CHARACTERISTICS:
       (A)   LENGTH: 33 nucleotides
       (B)   TYPE: nucleic acid
       (C)   STRANDEDNESS:  single
       (D)   TOPOLOGY: linear

(ii)   MOLECULE TYPE:  DNA

(xi)   SEQUENCE DESCRIPTION: SEQ ID NO: 140
ATCGATGACC TTACCCAART TRCGCGACCT RCG                33

(2)  INFORMATION FOR SEQ ID NO: 141

(i)    SEQUENCE CHARACTERISTICS:

(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 141
CCCCATGAGR TCGGCGAAGC CGCAYGTRAG GGT 33

(2) INFORMATION FOR SEQ ID NO: 142
(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 142
GCCYCCWARR GGGGCGCCGA CGAGCGGWAT RTA 33

(2) INFORMATION FOR SEQ ID NO: 143

(i) SEQUENCE CHARACTERISTICS:
(A) LENGTH: 33 nucleotides
(B) TYPE: nucleic acid

(C) STRANDEDNESS: single
(D) TOPOLOGY: linear

(ii) MOLECULE TYPE: DNA
(xi) SEQUENCE DESCRIPTION: SEQ ID NO: 143
AACCCGGACR CCRTGYGCCA RGGCCCTGGC AGC 33

(2) INFORMATION FOR SEQ ID NO: 144

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 144
RTTCCCTGTT GCATAGTTCA CGCCGTCYTC CAG 33

(2) INFORMATION FOR SEQ ID NO: 145

(i) SEQUENCE CHARACTERISTICS:
(A) LENGTH: 33 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 145
CARRAGGAAG AKAGAGAAAG AGCAACCRGG MAR 33

(2) INFORMATION FOR SEQ ID NO: 146

(i) SEQUENCE CHARACTERISTICS:
(A) LENGTH: 20 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 146
AGGCATAGGA CCCGTGTCTT 20

(2) INFORMATION FOR SEQ ID NO: 147

(i) SEQUENCE CHARACTERISTICS:
(A) LENGTH: 20 nucleotides
(B) TYPE: nucleic acid
(C) STRANDEDNESS: single
(D) TOPOLOGY: linear

(ii) MOLECULE TYPE: DNA

(xi) SEQUENCE DESCRIPTION: SEQ ID NO: 147
CTTCTTTGGA GAAAGTGGTG 20

## Description

This application is a continuation-in-part of U.S. Serial No. 07/697,326 entitled "Polynucleotide Probes Useful for Screening for Hepatitis C Virus, filed May 8, 1991.

### Technical Field

The invention relates to compositions and methods for the detection and treatment of hepatitis C virus, (HCV) infection, formerly referred to as blood-borne non-A, non-B hepatitis virus (NANBV) infection. More specifically, embodiments of the present invention feature compositions and methods for the detection of HCV, and for the development of vaccines for the prophylactic treatment of infections of HCV, and development of antibody products for conveying passive immunity to HCV.

### Background of the Invention

The prototype isolate of HCV was characterized in U.S. Patent Application Serial No. 122,714 (See also EPO Publication No. 318,216). As used herein, the term "HCV" includes new isolates of the same viral species. The term "HCV-1" referred to in U.S. Patent Application Serial No. 122,714.

HCV is a transmissible disease distinguishable from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). HCV was first identified in individuals who had received blood transfusions.

The demand for sensitive, specific methods for screening and identifying carriers of HCV and HCV contaminated blood or blood products is significant. Post-transfusion hepatitis (PTH) occurs in approximately 10% of transfused patients, and HCV accounts for up to 90% of these cases. The disease frequently progresses to chronic liver damage (25-55%).

Patient care as well as the prevention of transmission of HCV by blood and blood products or by close personal contact require reliable screening, diagnostic and prognostic tools to detect nucleic acids, antigens and antibodies related to HCV.

Information in this application suggests the HCV has several genotypes. That is, the genetic information of the HCV virus may not be totally identical for all HCV, but encompasses groups with differing genetic information.

Genetic information is stored in thread-like molecules of DNA and RNA. DNA consists of covalently linked chains of deoxyribonucleotides and RNA consists of covalently linked chains of ribonucleotides. Each nucleotide is characterized by one of four bases: adenine (A), guanine (G), thymine (T), and cytosine (C). The bases are complementary in the sense that, due to the orientation of functional groups, certain base pairs attract and bond to each other through hydrogen bonding and π-stacking interactions. Adenine in one strand of DNA pairs with thymine in an opposing complementary strand. Guanine in one strand of DNA pairs with cytosine in an opposing complementary strand. In RNA, the thymine base is replaced by uracil (U) which pairs with adenine in an opposing complementary strand. The genetic code of living organism is carried in the sequence of base pairs. Living cells interpret, transcribe and translate the information of nucleic acid to make proteins and peptides.

The HCV genome is comprised of a single positive strand of RNA. The HCV genome possesses a continuous, translational open reading frame (ORF) that encodes a polyprotein of about 3,000 amino acids. In the ORF, the structural protein(s) appear to be encoded in approximately the first quarter of the N-terminus region, with the majority of the polyprotein responsible for non-structural proteins.

The HCV polyprotein comprises, from the amino terminus to the carboxy terminus, the nucleocapsid protein (C), the envelope protein (E), and the non-structural proteins (NS) 1, 2 (b), 3, 4 (b), and 5.

HCV of differing genotypes may encode for proteins which present an altered response to host immune systems. HCV of differing genotypes may be difficult to detect by immuno diagnostic techniques and nucleic acid probe techniques which are not specifically directed to such genotype.

Definitions for selected terms used in the application are set forth below to facilitate an understanding of the invention. The term "corresponding" means homologous to or complementary to a particular sequence of nucleic acid. As between nucleic acids and peptides, corresponding refers to amino acids of a peptide in an order derived from the sequence of a nucleic acid or its complement.

The term "non-naturally occurring nucleic acid" refers to a portion of genomic nucleic acid, cDNA, semisynthetic nucleic acid, or synthetic origin nucleic acid which, by virtue of its origin or manipulation: (1) is not associated with all of a nucleic acid with which it is associated in nature, (2) is linked to a nucleic acid or other chemical agent other than that to which it is linked in nature, or (3) does not occur in nature.

Similarly the term, "a non-naturally occurring peptide" refers to a portion of a large naturally occurring peptide or protein, or semi-synthetic or synthetic peptide, which by virtue of its origin or manipulation (1) is not associated with all of a peptide with which it is associated in nature, (2) is linked to peptides, functional groups or chemical agents other than that to which it is linked in nature, or (3) does not occur in nature.

The term "primer" refers to a nucleic acid which is capable of initiating the synthesis of a larger nucleic acid when placed under appropriate conditions. The primer will be completely or substantially complementary to a region of the nucleic acid to be copied. Thus, under conditions conducive to hybridization, the primer will anneal to a complementary region of a larger nucleic acid. Upon addition of suitable reactants, the primer is extended by the polymerizing agent to form a copy of the larger nucleic acid.

The term "binding pair" refers to any pair of molecules which exhibit mutual affinity or binding capacity. For the purposes of the present application, the term "ligand" will refer to one molecule of the binding pair, and the term "antiligand" or "receptor" or "target" will refer to the opposite molecule of the binding pair. For example, with respect to nucleic acids, a binding pair may comprise two complementary nucleic acids. One of the nucleic acids may be designated the ligand and the other strand is designated the antiligand receptor or target. The designation of ligand or antiligand is a matter of arbitrary convenience. Other binding pairs comprise, by way of example, antigens and antibodies, drugs and drug receptor sites and enzymes and enzyme substrates, to name a few.

The term "label" refers to a molecular moiety capable of detection including, by way of example, without limitation, radioactive isotopes, enzymes, luminescent agents, precipitating agents, and dyes.

The term "support" includes conventional supports such as filters and membranes as well as retrievable supports which can be substantially dispersed within a medium and removed or separated from the medium by immobilization, filtering, partitioning, or the like. The term "support means" refers to supports capable of being associated to nucleic acids, peptides or antibodies by binding partners, or covalent or noncovalent linkages.

A number of HCV strains and isolates have been identified. When compared with the sequence of the original isolate derived from the USA ("HCV-1"; see Q.-L. Choo et al. (1989) Science 244:359-362, Q.-L. Choo et al. (1990) Brit. Med. Bull. 46:423-441, Q.-L. Choo et al., Proc. Natl. Acad. Sci. 88:2451-2455 (1991), and E.P.O. Patent Publication No. 318,216, cited supra), it was found that a Japanese isolate ("HCV J1") differed significantly in both nucleotide and polypeptide sequence within the NS3 and NS4 regions. This conclusion was later extended to the NS5 and envelope (E1/S and E2/NS1) regions (see K. Takeuchi et al., J. Gen. Virol. (1990) 71:3027-3033, Y. Kubo, Nucl. Acids. Res. (1989) 17:10367-10372, and K. Takeuchi et al., Gene (1990) 91:287-291). The former group of isolates, originally identified in the United States, is termed "Genotype I" throughout the present disclosure, while the latter group of isolates, initially identified in Japan, is termed "Genotype II" herein.

### Brief Description of the Invention

The present invention features compositions of matter comprising nucleic acids and peptides corresponding to the HCV viral genome which define different genotypes. The present invention also features methods of using the compositions corresponding to sequences of the HCV viral genome which define different genotypes described herein.

### A. Nucleic acid compositions

The nucleic acid of the present invention, corresponding to the HCV viral genome which define different genotypes, have utility as probes in nucleic acid hybridization assays, as primers for reactions involving the synthesis of nucleic acid, as binding partners for separating HCV viral nucleic acid from other constituents which may be present, and as anti-sense nucleic acid for preventing the transcription or translation of viral nucleic acid.

One embodiment of the present invention features a composition comprising a non-naturally occurring nucleic acid having a nucleic acid sequence of at least eight nucleotides corresponding to a non-HCV-1 nucleotide sequence of the hepatitis C viral genome. Preferably, the nucleotide sequence is selected from a sequence present in at least one region consisting of the NS5 region, envelope 1 region, 5'UT region, and the core region.

Preferably, with respect to sequences which correspond to the NS5 region, the sequence is selected from a sequence within a sequence numbered 2-22. The sequence numbered 1 corresponds to HCV-1. Sequences numbered 1-22 are defined in the Sequence Listing of the application.

Preferably, with respect to sequences corresponding to the envelope 1 region, the sequence is selected from a sequence within sequences numbered 24-32. Sequence No. 23 corresponds to HCV-1. Sequences numbered 23-32 are set forth in the Sequence Listing of the application.

Preferably, with respect to the sequences which correspond to the 5'UT regions, the sequence is selected from a sequence within sequences numbered 34-51. Sequence No. 33 corresponds to HCV-1. Sequence No. 33-51 are set forth in the Sequence Listing of this application.

Preferably, with respect to the sequences which correspond to the core region, the sequence is selected from a sequence within the sequences numbered 53-66. Sequence No. 52 corresponds to HCV-1. Sequences 52-66 are set forth in the Sequence Listing of this application.

The compositions of the present invention form hybridization products with nucleic acid corresponding to different genotypes of HCV.

HCV has at least five genotypes, which will be referred to in this application by the designations GI-GV. The first genotype, GI, is exemplified by sequences numbered 1-6, 23-25, 33-38 and 52-57. The second genotype, GII, is exemplified by the sequences numbered 7-12, 26-28, 39-45 and 58-64. The third genotype, GIII, is exemplified by sequences numbered 13-17, 32, 46-47 and 65-66. The fourth genotype, GIV, is exemplified by sequences numbered 20-22, and 29-31 and 48-49. The fifth genotype, GV, is exemplified by sequences numbered 18, 19, 50 and 51.

One embodiment of the present invention features compositions comprising a nucleic acid having a sequence corresponding to one or more sequences which exemplify a genotype of HCV.

### B. Method of forming a Hybridization Product

Embodiments of the present invention also feature a method of forming a hybridization product with nucleic acid having a sequence corresponding to HCV nucleic acid. One method comprises the steps of placing a non-naturally occurring nucleic acid having a non-HCV-1 sequence corresponding to HCV nucleic acid under conditions in which hybridization may occur. The non-naturally occurring nucleic acid is capable of forming a hybridization product with HCV nucleic acid, under hybridization conditions. The method further comprises the step of imposing hybridization conditions to form a hybridization product in the presence of nucleic acid corresponding to a region of the HCV genome.

The formation of a hybridization product has utility for detecting the presence of one or more genotypes of HCV. Preferably, the non-naturally occurring nucleic acid forms a hybridization product with nucleic acid of HCV in one or more regions comprising the NS5 region, envelope 1 region, 5'UT region and the core region. To detect the hybridization product, it is useful to associate the non-naturally occurring nucleic acid with a label. The formation of the hybridization product is detected by separating the hybridization product from labeled non-naturally occurring nucleic acid, which has not formed a hybridization product.

The formation of a hybridization product has utility as a means of separating one or more genotypes of HCV nucleic acid from other constituents potentially present. For such applications, it is useful to associate the non-naturally occurring nucleic acid with a support for separating the resultant hybridization product from the the other constituents.

Nucleic acid "sandwich assays" employ one nucleic acid associated with a label and a second nucleic acid associated with a support. An embodiment of the present invention features a sandwich assay comprising two nucleic acids, both have sequences which correspond to HCV nucleic acids; however, at least one non-naturally occurring nucleic acid has a sequence corresponding to non-HCV-1 HCV nucleic acid. At least one nucleic acid is capable of associating with a label, and the other is capable of associating with a support. The support associated non-naturally occurring nucleic acid is used to separate the hybridization products which include an HCV nucleic acid and the non-naturally occurring nucleic acid having a non-HCV-1 sequence.

One embodiment of the present invention features a method of detecting one or more genotypes of HCV. The method comprises the steps of placing a non-naturally occurring nucleic acid under conditions which hybridization may occur. The non-naturally occurring nucleic acid is capable of forming a hybridization product with nucleic acid from one or more genotypes of HCV. The first genotype, GI, is exemplified by sequences numbered 1-6, 23-25, 33-38 and 52-57. The second genotype, GII, is exemplified by the sequences numbered 7-12, 26-28, 39-45 and 58-64. The third genotype, GIII, is exemplified by sequences numbered 13-17, 32, 46-47 and 65-66. The fourth genotype, GIV, is exemplified sequences numbered 20-22 and 29-31. The fifth genotype, GV, is exemplified by sequences numbered 18, 19, 50 and 51.

The hybridization product of HCV nucleic acid with a non-naturally occurring nucleic acid having non-HCV-1 sequence corresponding to sequences within the HCV genome has utility for priming a reaction for the synthesis of nucleic acid.

The hybridization product of HCV nucleic acid with a non-naturally occurring nucleic acid having a sequence corresponding to a particular genotype of HCV has utility for priming a reaction for the synthesis of nucleic acid of such genotype. In one embodiment, the synthesized nucleic acid is indicative of the presence of one or more genotypes of HCV.

The synthesis of nucleic acid may also facilitate cloning of the nucleic acid into expression vectors which synthesize viral proteins.

Embodiments of the present methods have utility as anti-sense agents for preventing the transcription or translation of viral nucleic acid. The formation of a hybridization product of a non-naturally occurring nucleic acid having sequences which correspond to a particular genotype of HCV genomic sequencing with HCV nucleic acid may block translation or transcription of such genotype. Therapeutic agents can be engineered to include all five genotypes for inclusivity.

### C. Peptide and antibody composition

A further embodiment of the present invention features a composition of matter comprising a non-naturally occurring peptide of three or more amino acids corresponding to a nucleic acid having a non-HCV-1 sequence. Preferably, the non-HCV-1 sequence corresponds with a sequence within one or more regions consisting of the NS5 region, the envelope 1 region, the 5'UT region, and the core region.

Preferably, with respect to peptides corresponding to a nucleic acid having a non-HCV-1 sequence of the NS5 region, the sequence is within sequences numbered 2-22. The sequence numbered 1 corresponds to HCV-1. Sequences numbered 1-22 are set forth in the Sequence Listing.

Preferably, with respect to peptides corresponding to a nucleic acid having a non-HCV-1 sequence of the envelope 1 region, the sequence is within sequences numbered 24-32. The sequence numbered 23 corresponds to HCV-1. Sequences numbered 23-32 are set forth in the Sequence Listing.

Preferably, with respect to peptides corresponding to a nucleic acid having a non-HCV-1 sequence directed to the core region, the sequence is within sequences numbered 53-66. Seguence numbered 52 corresponds to HCV-1. Sequences numbered 52-66 are set forth in the Sequence Listing.

The further embodiment of the present invention features peptide compositions corresponding to nucleic acid sequences of a genotype of HCV. The first genotype, GI, is exemplified by sequences numbered 1-6, 23-25, 33-38 and 52-57. The second genotype, GII, is exemplified by the sequences numbered 7-12, 26-28, 39-45 and 58-64. The third genotype, GIII, is exemplified by sequences numbered 13-17, 32, 46-47 and 65-66. The fourth genotype, GIV, is exemplified sequences numbered 20-22, 29-31, 48 and 49. The fifth genotype, GV, is exemplified by sequences numbered 18, 19, 50 and 51.

The non-naturally occurring peptides of the present invention are useful as a component of a vaccine. The sequence information of the present invention permits the design of vaccines which are inclusive for all or some of the different genotypes of HCV. Directing a vaccine to a particular genotype allows prophylactic treatment to be tailored to maximize the protection to those agents likely to be encountered. Directing a vaccine to more than one genotype allows the vaccine to be more inclusive.

The peptide compositions are also useful for the development of specific antibodies to the HCV proteins. One embodiment of the present invention features as a composition of matter, an antibody to peptides corresponding to a non-HCV-1 sequence of the HCV genome. Preferably, the non-HCV-1 sequence is selected from the sequence within a region consisting of the NS5 region, the envelope 1 region, and the core region. There are no peptides associated with the untranslated 5'UT region.

Preferably, with respect to antibodies directed to peptides of the NS5 region, the peptide corresponds to a sequence within sequences numbered 2-22. Preferably, with respect to antibodies directed to a peptide corresponding to the envelope 1 region, the peptide corresponds to a sequence within sequences numbered 24-32. Preferably, with respect to the antibodies directed to peptides corresponding to the core region, the peptide corresponds to a sequence within sequences numbered 53-66.

Antibodies directed to peptides which reflect a particular genotype have utility for the detection of such genotypes of HCV and therapeutic agents.

One embodiment of the present invention features an antibody directed to a peptide corresponding to nucleic acid having sequences of a particular genotype. The first genotype, GI, is exemplified by sequences numbered 1-6, 23-25, 33-38 and 52-57. The second genotype, GII, is exemplified by the sequences numbered 7-12, 26-28, 39-45 and 58-64. The third genotype, GIII, is exemplified by sequences numbered 13-17, 32, 46-47 and 65-66. The fourth genotype, GIV, is exemplified sequences numbered 20-22, 29-31, 48 and 49. The fifth genotype, GV, is exemplified by sequences numbered 18, 19, 50 and 51.

Individuals skilled in the art will readily recognize that the compositions of the present invention can be packaged with instructions for use in the form of a kit for performing nucleic acid hybridizations or immunochemical reactions.

The present invention is further described in the following figures which illustrate sequences demonstrating genotypes of HCV. The sequences are designated by numerals 1-145, which numerals and sequences are consistent with the numerals and sequences set forth in the Sequence Listing. Sequences 146 and 147 facilitate the discussion of an assay which numerals and sequences are consistent with the numerals and sequences set forth in-the Sequence Listing.

### Brief Description of the Figures and Sequence Listing

Figure 1 depicts schematically the genetic organization of HCV;
Figure 2 sets forth nucleic acid sequences numbered 1-22 which sequences are derived from the NS5 region of the HCV viral genome;
Figure 3 sets forth nucleic acid sequences numbered 23-32 which sequences are derived from the envelope 1 region of the HCV viral genome;
Figure 4 sets forth nucleic acid sequences numbered 33-51 which sequences are derived from the 5'UT region of the HCV viral genome; and,
Figure 5 sets forth nucleic acid sequences numbered 52-66 which sequences are derived from the core region of the HCV viral genome.

The Sequence Listing sets forth the sequences of sequences numbered 1-147.

### Detailed Description of the Invention

The present invention will be described in detail as as nucleic acid having sequences corresponding to the HCV genome and related peptides and binding partners, for diagnostic and therapeutic applications.

The practice of the present invention will employ, unless otherwise indicated, conventional techniques of chemistry, molecular biology, microbiology, recombinant DNA, and immunology, which are within the skill of the art. Such techniques are explained fully in the literature. See e.g., Maniatis, Fitsch & Sambrook, Molecular Cloning; A Laboratory Manual (1982); DNA Cloning, Volumes I and II (D.N Glover ed. 1985); Oligonucleotide Synthesis (M.J. Gait ed, 1984); Nucleic Acid Hybridization (B.D. Hames & S.J. Higgins eds. 1984); the series, Methods in Enzymology (Academic Press, Inc.), particularly Vol. 154 and Vol. 155 (Wu and Grossman, eds.).

The cDNA libraries are derived from nucleic acid sequences present in the plasma of an HCV-infected chimpanzee. The construction of one of these libraries, the "c" library (ATCC No. 40394), is described in PCT Pub. No. WO90/14436. The sequences of the library relevant to the present invention are set forth herein as sequence numbers 1, 23, 33 and 52.

Nucleic acids isolated or synthesized in accordance with features of the present invention are useful, by way of example without limitation as probes, primers, anti-sense genes and for developing expression systems for the synthesis of peptides corresponding to such sequences.

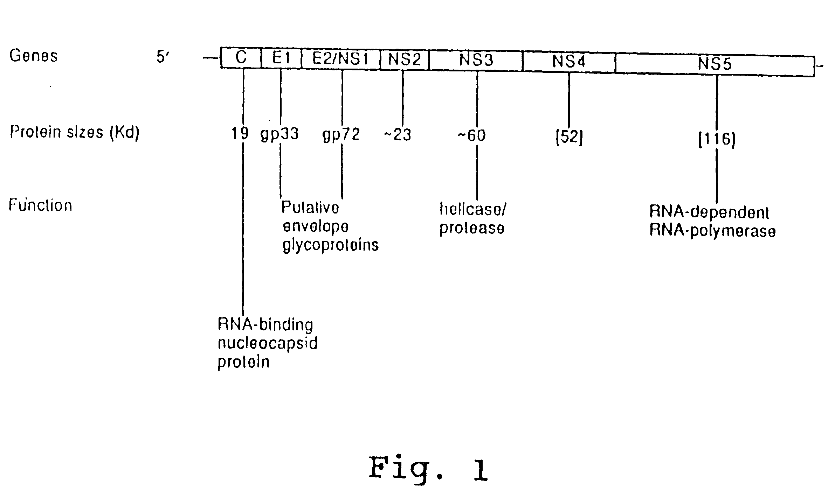

The nucleic acid sequences described define genotypes of HCV with respect to four regions of the viral genome. Figure 1 depicts schematically the organization of HCV. The four regions of particular interest are the NS5 region, the envelope 1 region, the 5'UT region and the core region.

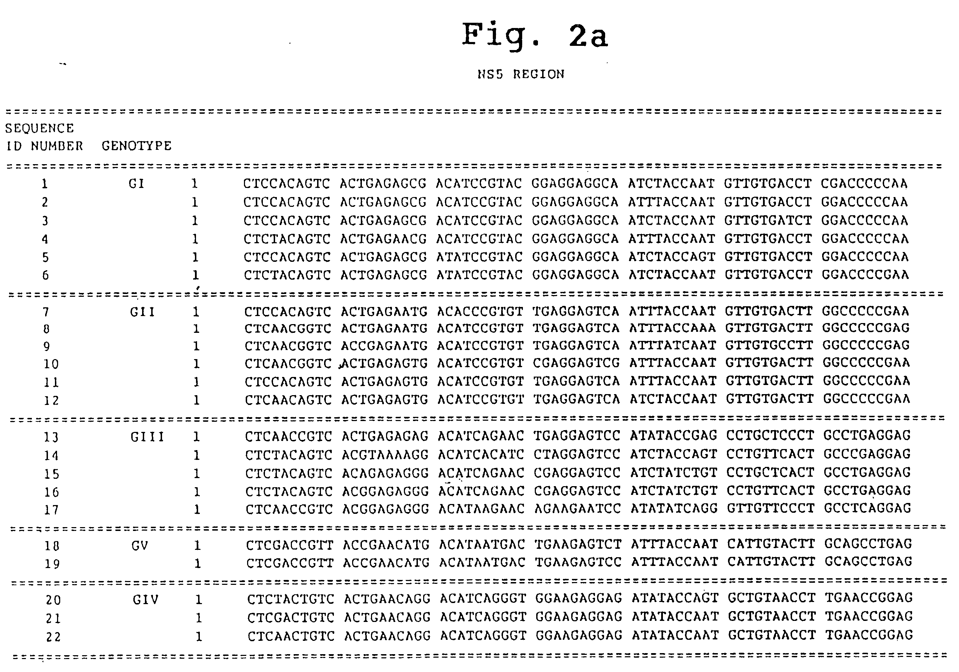

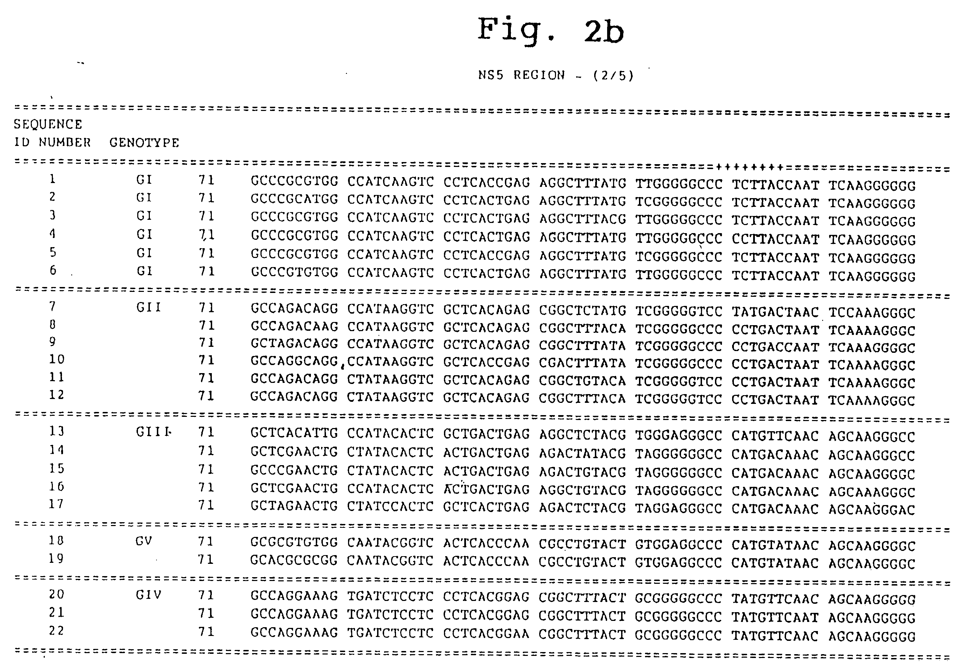

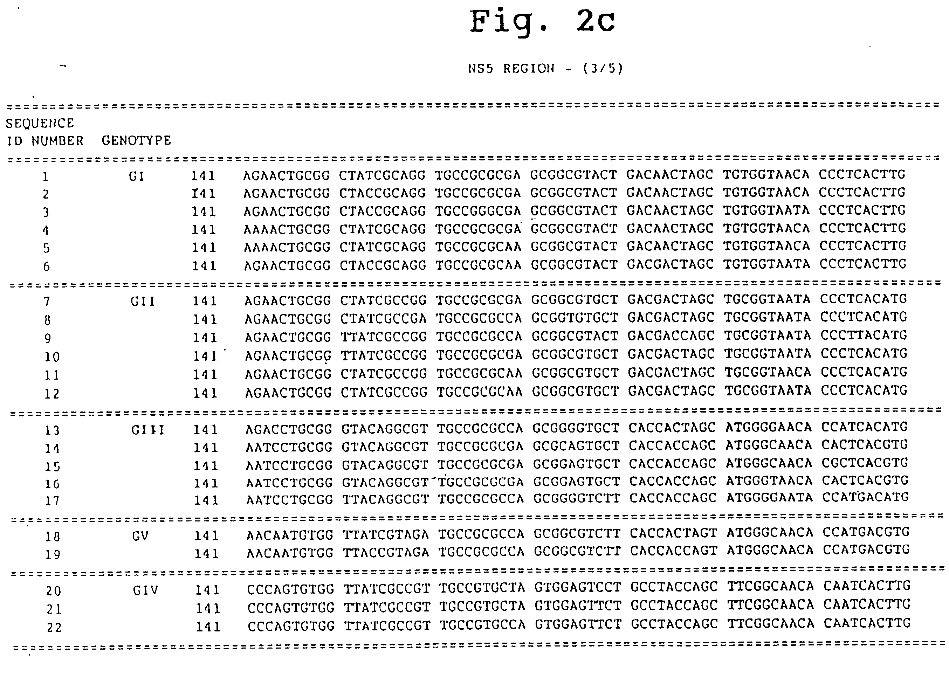

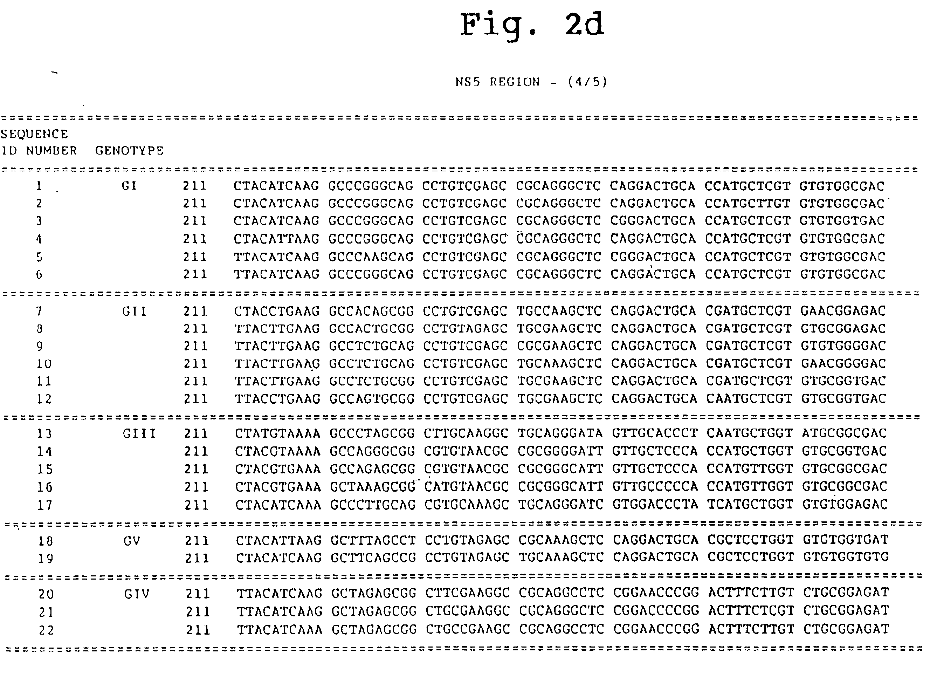

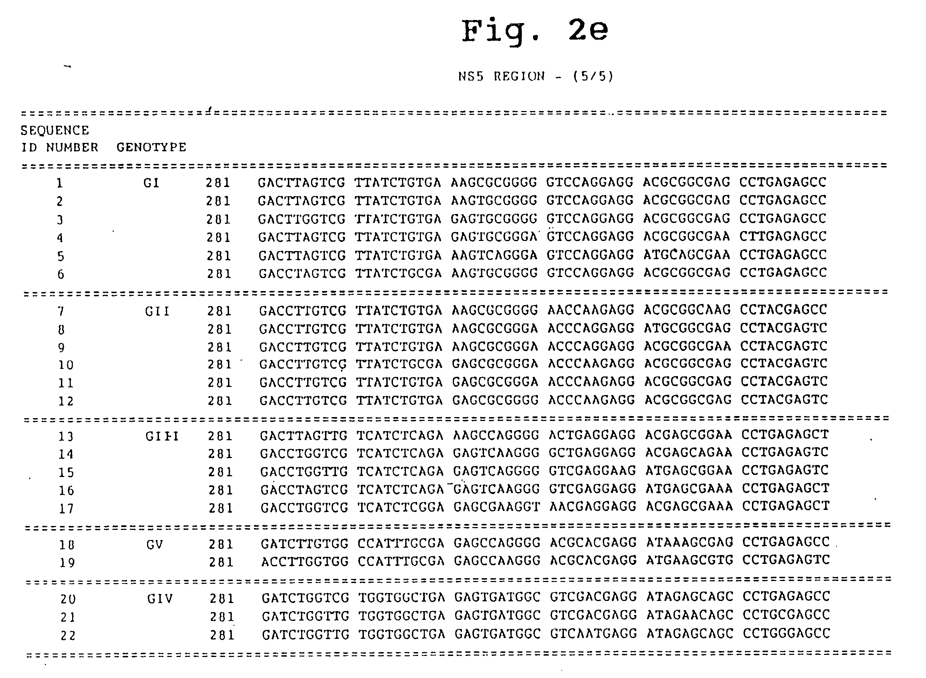

The sequences set forth in the present application as sequences numbered 1-22 suggest at least five genotypes in the NS5 region. Sequences numbered 1-22 are depicted in Figure 2 as well as the Sequence Listing. Each sequence numbered 1-22 is derived from nucleic acid having 340 nucleotides from the NS5 region.

The five genotypes are defined by groupings of the sequences defined by sequence numbered 1-22. For convenience, in the present application, the different genotypes will be assigned roman numerals and the letter "G".

The first genotype (GI) is exemplified by sequences within sequences numbered 1-6. A second genotype (GII) is exemplified by sequences within sequences numbered 7-12. A third genotype (GIII) is exemplified by the sequences within sequences numbered 13-17. A fourth genotype (GIV) is exemplified by sequences within sequences numbered 20-22. A fifth genotype (GV) is exemplified by sequences within sequences numbered 18 and 19.

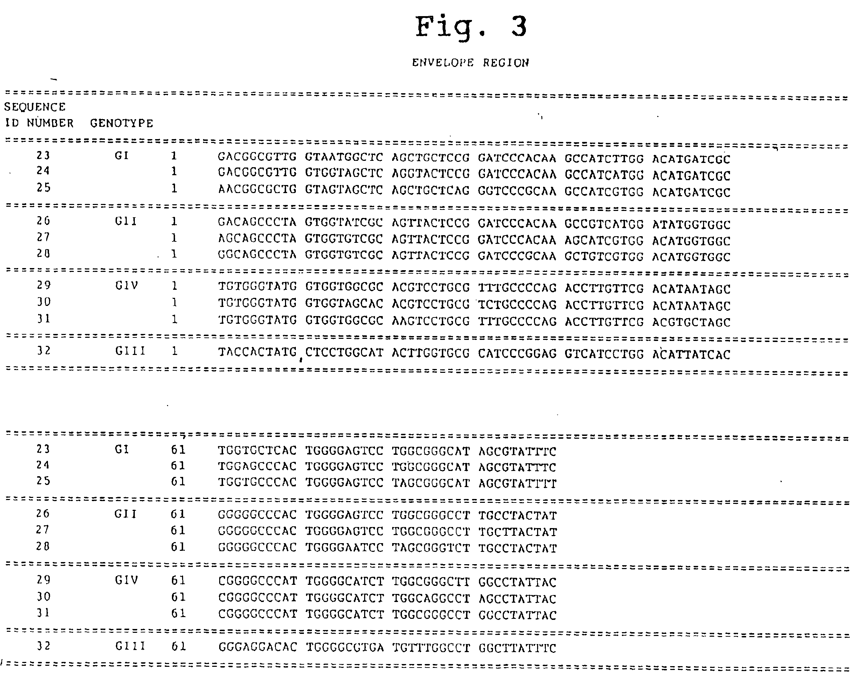

The sequences set forth in the present application as sequences numbered 23-32 suggest at least four genotypes in the envelope 1 region of HCV. Sequences numbered 23-32 are depicted in Figure 3 as well as in the Sequence Listing. Each sequence numbered 23-32 is - derived from nucleic acid having 100 nucleotides from the envelope 1 region.

A first envelope 1 genotype group (GI) is exemplified by the sequences within the sequences numbered 23-25. A second envelope 1 genotype (GII) region is exemplified by sequences within sequences numbered 26-28. A third envelope 1 genotype (GIII) is exemplified by the sequences within sequences numbered 32. A fourth envelope 1 genotype (GIV) is exemplified by the sequences within sequence numbered 29-31.

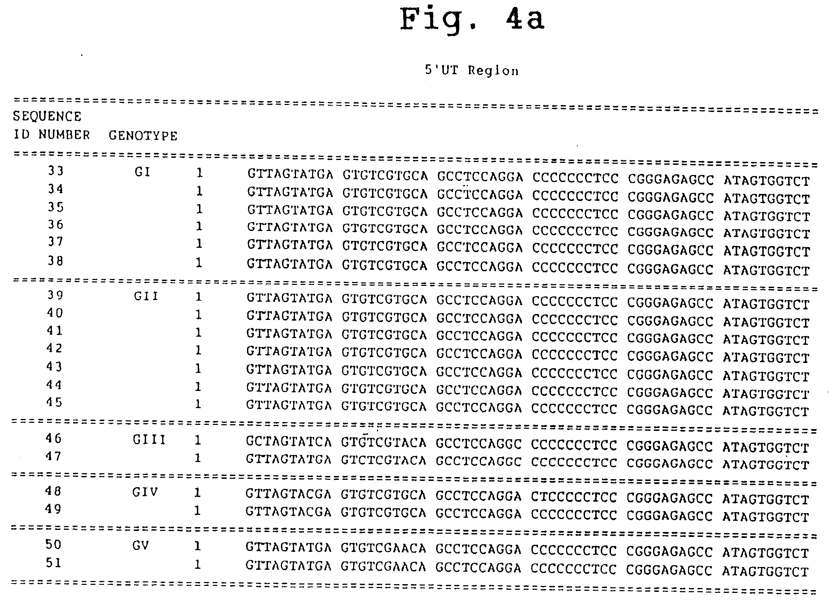

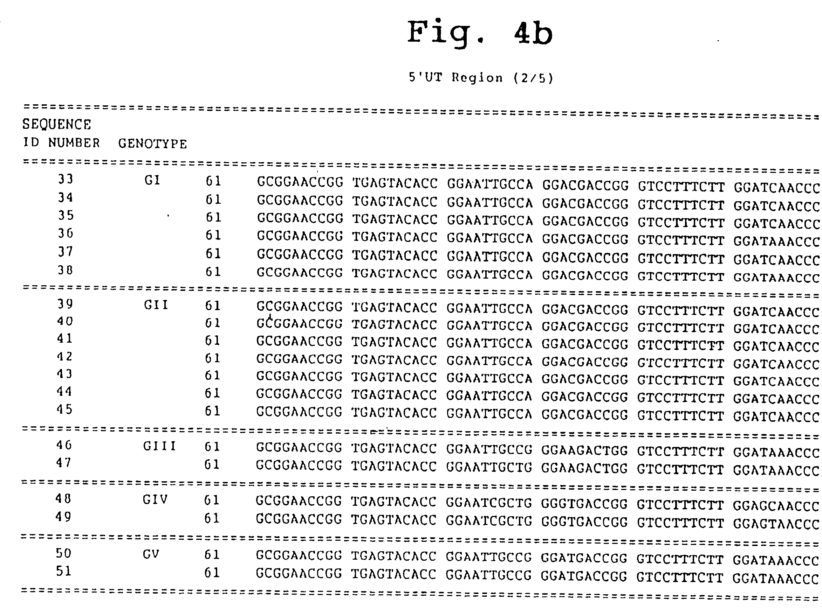

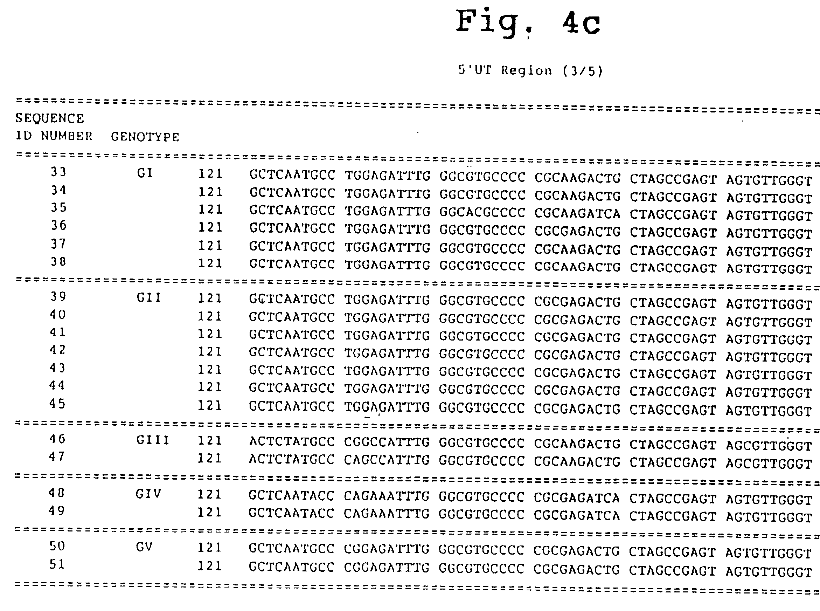

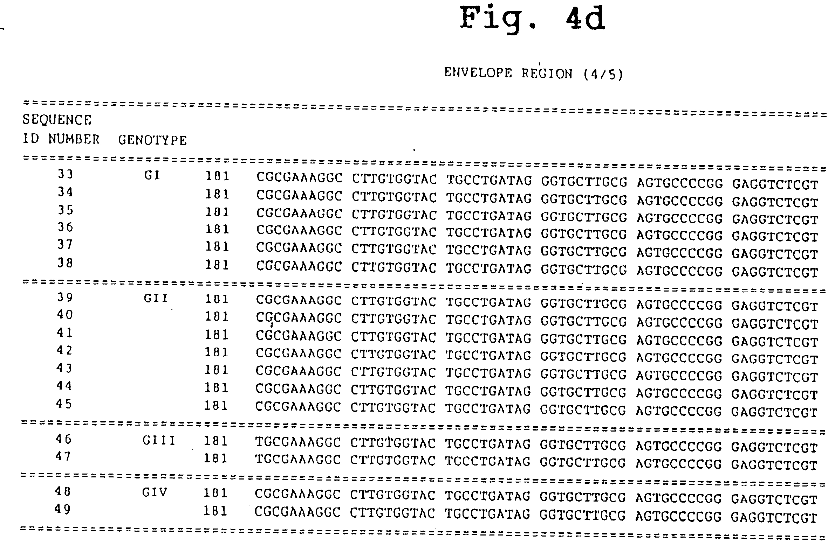

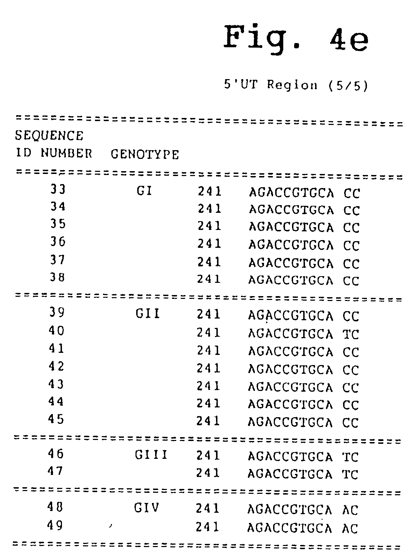

The sequences set forth in the present application as sequences numbered 33-51 suggest at least three genotypes in the 5'UT region of HCV. Sequences numbered 33-51 are depicted in Figure 4 as well as in the Sequence Listing. Each sequence numbered 33-51 is derived from the nucleic acid having 252 nucleotides from the 5'UT region, although sequences 50 and 51 are somewhat shorter at approximately 180 nucleotides.

The first 5'UT genotype (GI) is exemplified by the sequences within sequences numbered 33-38. A second 5'UT genotype (GII) is exemplified by the sequences within sequences numbered 39-45. A third 5'UT genotype (GIII) is exemplified by the sequences within sequences numbered 46-47. A fourth 5'UT genotype (GIV) is exemplified by sequences within sequences humbered 48 and 49. A fifth 5'UT genotype (GV) is exemplified by sequences within sequences numbered 50 and 51.

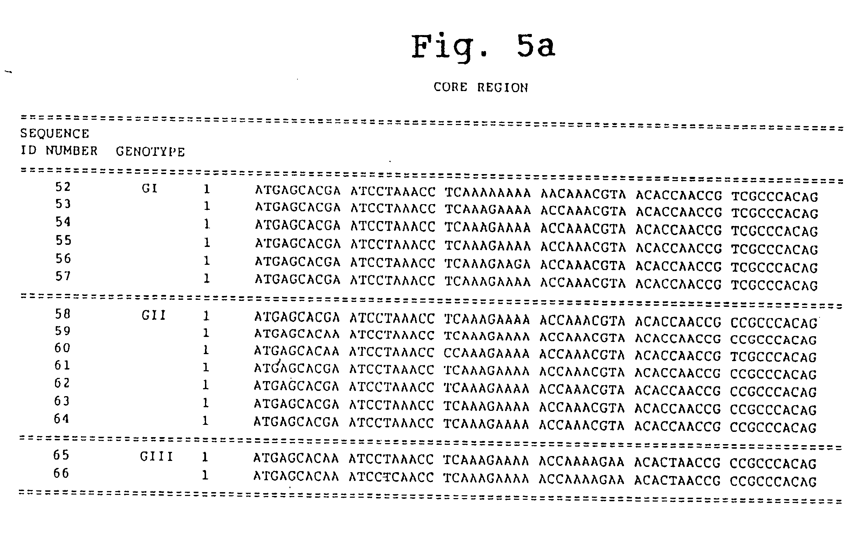

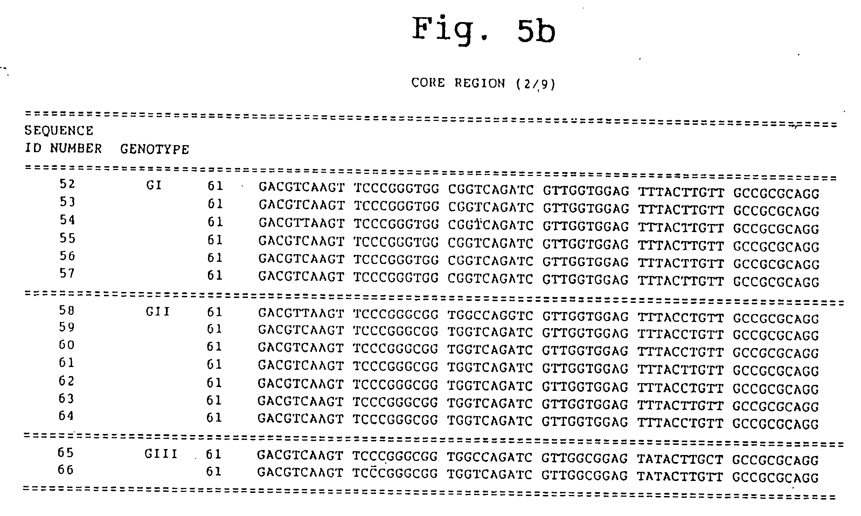

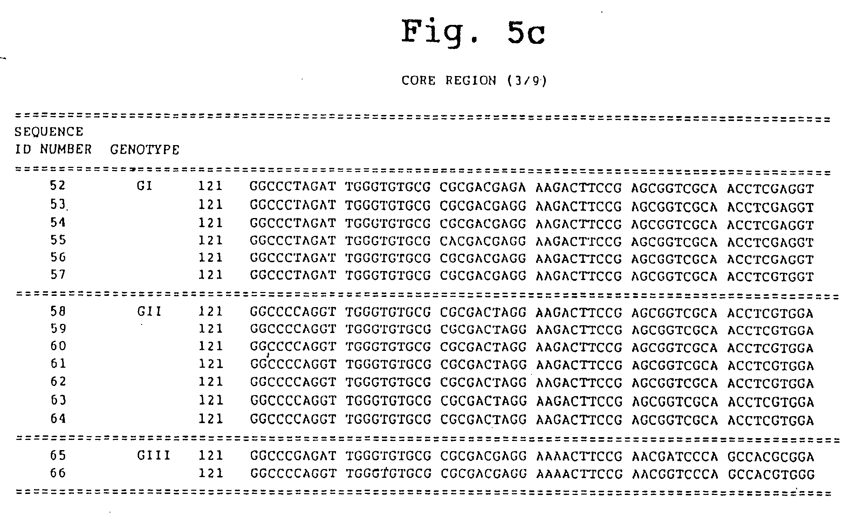

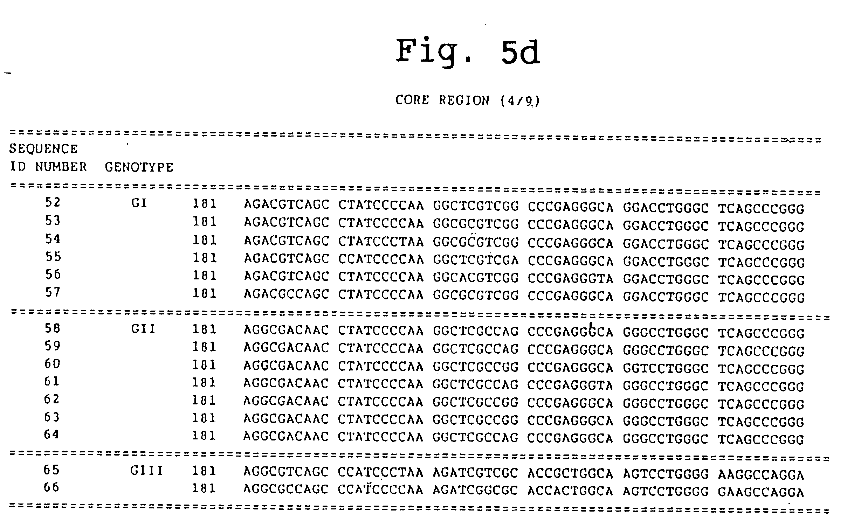

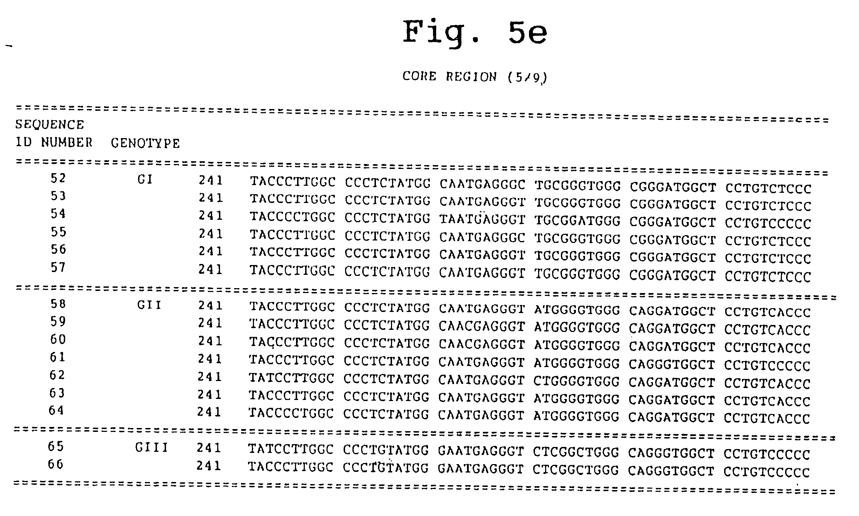

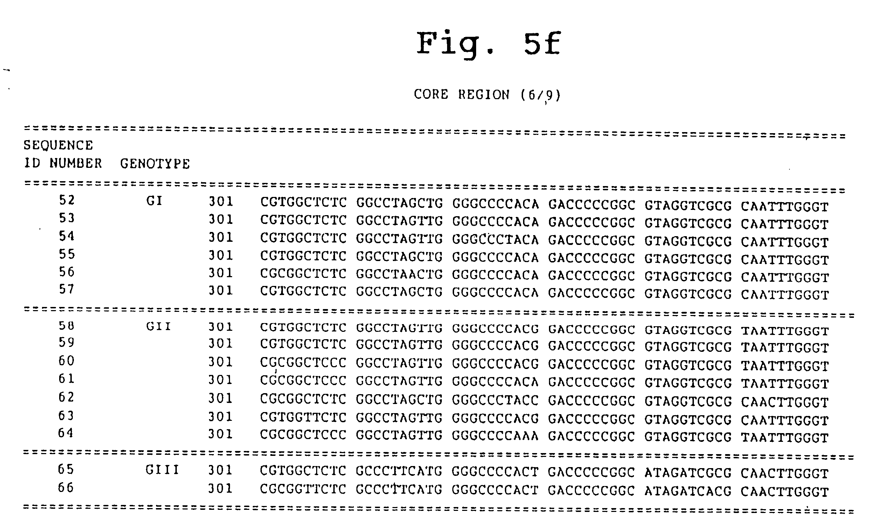

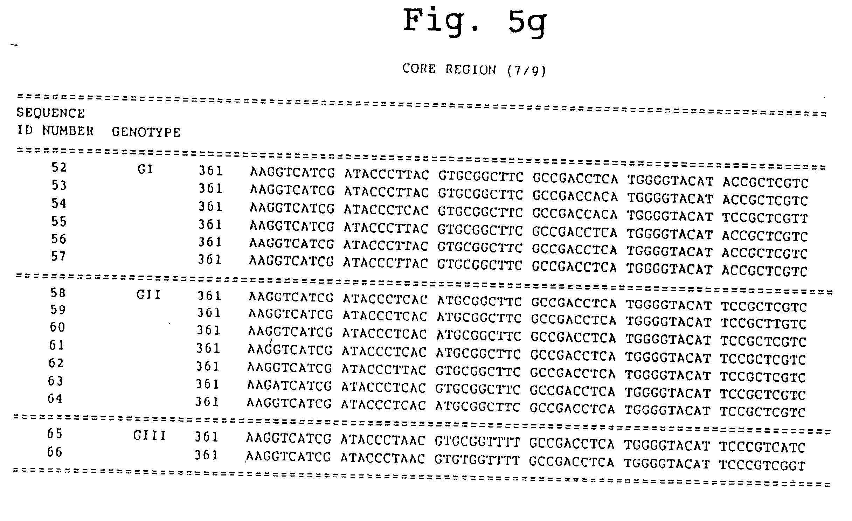

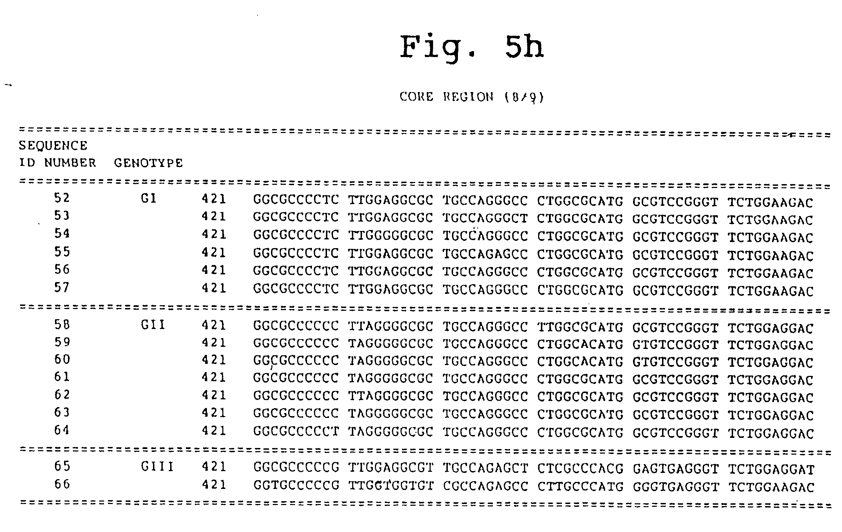

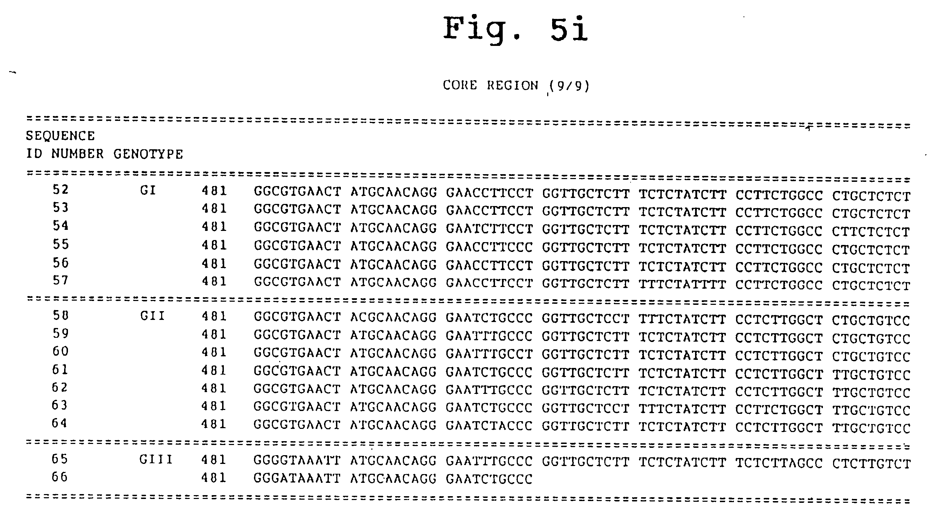

The sequences numbered 48-62 suggest at least three genotypes in the core region of HCV. The sequences numbered 52-66 are depicted in Figure 5 as well as in the Sequence Listing.

The first core region genotype (GI) is exemplified by the sequences within sequences numbered 52-57. The second core region genotype (GII) is exemplified by sequences within sequences numbered 58-64. The third core region genotype (GIII) is exemplified by sequences within sequences numbered 65 and 66. Sequences numbered 52-65 are comprised of 549 nucleotides. Sequence numbered 66 is comprised of 510 nucleotides.

The various genotypes described with respect to each region are consistent. That is, HCV having features of the first genotype with respect to the NS5 region will substantially conform to features of the first genotype of the envelope 1 region, the 5'UT region and the core region.

Nucleic acid isolated or synthesized in accordance with the sequences set forth in sequence numbers 1-66 are useful as probes, primers, capture ligands and anti-sense agents. As probes, primers, capture ligands and anti-sense agents, the nucleic acid wil normally comprise approximately eight or more nucleotides for specificity as well as the ability to form stable hybridization products.

### Probes

A nucleic acid isolated or synthesized in accordance with a sequence defining a particular genotype of a region of the HCV genome can be used as a probe to detect such genotype or used in combination with other nucleic acid probes to detect substantially all genotypes of HCV.

With the sequence information set forth in the present application, sequences of eight or more nucleotides are identified which provide the desired inclusivity and exclusivity with respect to various genotypes within HCV, and extraneous nucleic acid sequences likely to be encountered during hybridization conditions.

Individuals skilled in the art will readily recognize that the nucleic acid sequences, for use as probes, can be provided with a label to facilitate detection of a hybridization product.

### Capture Ligand

For use as a capture ligand, the nucleic acid selected in the manner described above with respect to probes, can be readily associated with supports. The manner in which nucleic acid is associated with supports is well known. Nucleic acid having sequences corresponding to a sequence within sequences numbered 1-66 have utility to separate viral nucleic acid of one genotype from the nucleic acid of HCV of a different genotype. Nucleic acid isolated or synthesized in accordance with sequences within sequences numbered 1-66, used in combinations, have utility to capture substantially all nucleic acid of all HCV genotypes.

### Primers

Nucleic acid isolated or synthesized in accordance with the sequences described herein have utility as primers for the amplification of HCV sequences. With respect to polymerase chain reaction (PCR) techniques, nucleic acid sequences of eight or more nucleotides corresponding to one or more sequences of sequences numbered 1-66 have utility in conjunction with suitable enzymes and reagents to create copies of the viral nucleic acid. A plurality of primers having different sequences corresponding to more than one genotype can be used to create copies of viral nucleic acid for such genotypes.

The copies can be used in diagnostic assays to detect HCV virus. The copies can also be incorporated into cloning and expression vectors to generate polypeptides corresponding to the nucleic acid synthesized by PCR, as will be described in greater detail below.

### Anti-sense

Nucleic acid isolated or synthesized in accordance with the sequences described herein have utility as anti-sense genes to prevent the expression of HCV.

Nucleic acid corresponding to a genotype of HCV is loaded into a suitable carrier such as a liposome for introduction into a cell infected with HCV. A nucleic acid having eight or more nucleotides is capable of binding to viral nucleic acid or viral messenger RNA. Preferably, the anti-sense nucleic acid is comprised of 30 or more nucleotides to provide necessary stability of a hybridization product of viral nucleic acid or viral messenger RNA. Methods for loading anti-sense nucleic acid is known in the art as exemplified by U.S. Patent 4,241,046 issued December 23, 1980 to Papahadjopoulos et al.

### Peptide Synthesis

Nucleic acid isolated or synthesized in accordance with the sequences described herein have utility to generate peptides. The sequences exemplified by sequences numbered 1-32 and 52-66 can be cloned into suitable vectors or used to isolate nucleic acid. The isolated nucleic acid is combined with suitable DNA linkers and cloned into a suitable vector. The vector can be used to transform a suitable host organism such as E. coli and the peptide encoded by the sequences isolated.

Molecular cloning techniques are described in the text Molecular Cloning: A Laboratory Manual, Maniatis et al., Coldspring Harbor Laboratory (1982).

The isolated peptide has utility as an antigenic substance for the development of vaccines and antibodies directed to the particular genotype of HCV.

### Vaccines and Antibodies

The peptide materials of the present invention have utility for the development of antibodies and vaccines.

The availability of cDNA sequences, or nucleotide sequences derived therefrom (including segments and modifications of the sequence), permits the construction of expression vectors encoding antigenically active regions of the peptide encoded in either strand. The antigenically active regions may be derived from the NS5 region, envelope 1 regions, and the core region.

Fragments encoding the desired peptides are derived from the cDNA clones using conventional restriction digestion or by synthetic methods, and are ligated into vectors which may, for example, contain portions of fusion sequences such as beta galactosidase or superoxide dismutase (SOD), preferably SOD. Methods and vectors which are useful for the production of polypeptides which contain fusion sequences of SOD are described in European Patent Office Publication number 0196056, published October 1, 1986.

Any desired portion of the HCV cDNA containing an open reading frame, in either sense strand, can be obtained as a recombinant peptide, such as a mature or fusion protein; alternatively, a peptide encoded in the CDNA can be provided by chemical synthesis.

The DNA encoding the desired peptide, whether in fused or mature form, and whether or not containing a signal sequence to permit secretion, may be ligated into expression vectors suitable for any convenient host. Both eukaryotic and prokaryotic host systems are presently used in forming recombinant peptides. The peptide is then isolated from lysed cells or from the culture medium and purified to the extent needed for its intended use. Purification may be by techniques known in the art, for example, differential extraction, salt fractionation, chromatography on ion exchange resins, affinity chromatography, centrifugation, and the like. See, for example, Methods in Enzymology for a variety of methods for purifying proteins. Such peptides can be used as diagnostics, or those which give rise to neutralizing antibodies may be formulated into vaccines. Antibodies raised against these peptides can also be used as diagnostics, or for passive immunotherapy or for isolating and identifying HCV.

An antigenic region of a peptide is generally relatively small-typically 8 to 10 amino acids or less in length. Fragments of as few as 5 amino acids may characterize an antigenic region. These segments may correspond to NS5 region, envelope 1 region, and the core region of the HCV genome. The 5'UT region is not known to be translated. Accordingly, using the cDNAs of such regions, DNAs encoding short segments of HCV peptides corresponding to such regions can be expressed recombinantly either as fusion proteins, or as isolated peptides. In addition, short amino acid sequences can be conveniently obtained by chemical synthesis. In instances wherein the synthesized peptide is correctly configured so as to provide the correct epitope, but is too small to be immunogenic, the peptide may be linked to a suitable carrier.

A number of techniques for obtaining such linkage are known in the art, including the formation of disulfide linkages using N-succinimidyl-3-(2-pyridylthio)propionate (SPDP) and succinimidyl 4-(N-maleimido-methyl)cyclohexane-l-carboxylate (SMCC) obtained from Pierce Company, Rockford, Illinois, (if the peptide lacks a sulfhydryl group, this can be provided by addition of a cysteine residue). These reagents create a disulfide linkage between themselves and peptide cysteine residues on one protein and an amide linkage through the epsilon-amino on a lysine, or other free amino group in the other. A variety of such disulfide/amide-forming agents are known. See, for example, Immun Rev (1982) 62:185. Other bifunctional coupling agents form a thioether rather than a disulfide linkage. Many of these thio-ether-forming agents are commercially available and include reactive esters of 6-maleimidocaprioc acid, 2-bromoacetic acid, 2-iodoacetic acid, 4-N-maleimido-methyl)cyclohexane-1-carboxylic acid, and the like. The carboxyl groups can be activated by combining them with succinimide or 1-hydroxyl-2 nitro-4-sulfonic acid, sodium salt. Additional methods of coupling antigens employs the rotavirus/"binding peptide" system described in EPO Pub. No. 259,149, the disclosure of which is incorporated herein by reference. The foregoing list is not meant to be exhaustive, and modifications of the named compounds can clearly be used.

Any carrier may be used which does not itself induce the production of antibodies harmful to the host. Suitable carriers are typically large, slowly metabolized macromolecules such as proteins; polysaccharides, such as latex functionalized Sepharose, agarose, cellulose, cellulose beads and the like; polymeric amino acids, such as polyglutamic acid, polylysine, and the like; amino acid copolymers; and inactive virus particles. Especially useful protein substrates are serum albumins, keyhole limpet hemocyanin, immunoglobulin molecules, thyroglobulin, ovalbumin, tetanus toxoid, and other proteins well known to those skilled in the art.

Peptides comprising HCV amino acid sequences encoding at least one viral epitope derived from the NS5, envelope 1, and core region are useful immunological reagents. The 5'UT region is not known to be translated. For example, peptides comprising such truncated sequences can be used as reagents in an immunoassay. These peptides also are candidate subunit antigens in compositions for antiserum production or vaccines. While the truncated sequences can be produced by various known treatments of native viral protein, it is generally preferred to make synthetic or recombinant peptides comprising HCV sequence. Peptides comprising these truncated HCV sequences can be made up entirely of HCV sequences (one or more epitopes, either contiguous or noncontiguous), or HCV sequences and heterologous sequences in a fusion protein. Useful heterologous sequences include sequences that provide for secretion from a recombinant host, enhance the immunological reactivity of the HCV epitope(s), or facilitate the coupling of the polypeptide to an immunoassay support or a vaccine carrier. See, E.G., EPO Pub. No. 116,201; U.S. Pat. No. 4,722,840; EPO Pub. No. 259,149; U.S. Pat. No. 4,629,783.

The size of peptides comprising the truncated HCV sequences can vary widely, the minimum size being a sequence of sufficient size to provide an HCV epitope, while the maximum size is not critical. For convenience, the maximum size usually is not substantially greater than that required to provide the desired HCV epitopes and function(s) of the heterologous sequence, if any. Typically, the truncated HCV amino acid sequence will range from about 5 to about 100 amino acids in length. More typically, however, the HCV sequence will be a maximum of about 50 amino acids in length, preferably a maximum of about 30 amino acids. It is usually desirable to select HCV sequences of at least about 10, 12 or 15 amino acids, up to a maximum of about 20 or 25 amino acids.

HCV amino acid sequences comprising epitopes can be identified in a number of ways. For example, the entire protein sequence corresponding to each of the NS5, envelope 1, and core regions can be screened by preparing a series of short peptides that together span the entire protein sequence of such regions. By starting with, for example, peptides of approximately 100 amino acids, it would be routine to test each peptide for the presence of epitope(s) showing a desired reactivity, and then testing progressively smaller and overlapping fragments from an identified peptides of 100 amino acids to map the epitope of interest. Screening such peptides in an immunoassay is within the skill of the art. It is also known to carry out a computer analysis of a protein sequence to identify potential epitopes, and then prepare peptides comprising the identified regions for screening.

The immunogenicity of the epitopes of HCV may also be enhanced by preparing them in mammalian or yeast systems fused with or assembled with particle-forming proteins such as, for example, that associated with hepatitis B surface antigen. See, e.g., US 4,722,840. Constructs wherein the HCV epitope is linked directly to the particle-forming protein coding sequences produce hybrids which are immunogenic with respect to the HCV epitope. In addition, all of the vectors prepared include epitopes specific to HBV, having various degrees of immunogenicity, such as, for example, the pre-S peptide. Thus, particles constructed from particle forming protein which include HCV sequences are immunogenic with respect to HCV and HBV.

Hepatitis surface antigen (HBSAg) has been shown to be formed and assembled into particles in S. cerevisiae (P. Valenzuela et al. (1982)), as well as in, for example, mammalian cells (P. Valenzuela et al. 1984)). The formation of such particles has been shown to enhance the immunogenicity of the monomer subunit. The constructs may also include the immunodominant epitope of HBSAg, comprising the 55 amino acids of the presurface (pre-S) region. Neurath et al. (1984). Constructs of the pre-S-HBSAg particle expressible in yeast are disclosed in EPO 174,444, published March 19, 1986; hybrids including heterologous viral sequences for yeast expression are disclosed in EPO 175,261, published March 26, 1966. These constructs may also be expressed in mammalian cells such as Chinese hamster ovary (CHO) cells using an SV40-dihydrofolate reductase vector (Michelle et al. (1984)).

In addition, portions of the particle-forming protein coding sequence may be replaced with codons encoding an HCV epitope. In this replacement, regions which are not required to mediate the aggregation of the units to form immunogenic particles in yeast of mammals can be deleted, thus eliminating additional HBV antigenic sites from competition with the HCV epitope.

### Vaccines

Vaccines may be prepared from one or more immunogenic peptides derived from HCV. The observed homology between HCV and Flaviviruses provides information concerning the peptides which are likely to be most effective as vaccines, as well as the regions of the genome in which they are encoded.

Multivalent vaccines against HCV may be comprised of one or more epitopes from one or more proteins derived from the NS5, envelope 1, and core regions. In particular, vaccines are contemplated comprising one or more HCV proteins or subunit antigens derived from the NS5, envelope 1, and core regions. The 5'UT region is not known to be translated.

The preparation of vaccines which contain an immunogenic peptide as an active ingredient, is known to one skilled in the art. Typically, such vaccines are prepared as injectables, either as liquid solutions or suspensions; solid forms suitable for solution in, or suspension in, liquid prior to injection may also be prepared. The preparation may also be emulsified, or the protein encapsulated in liposomes. The active immunogenic ingredients are often mixed with excipients which are pharmaceutically acceptable and compatible with the active ingredient. Suitable excipients are, for example, water, saline, dextrose, glycerol, ethanol, or the like and combinations thereof. In addition, if desired, the vaccine may contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide, N-acetyl-muramyl-L-theronyl-D- isoglutamine (thr-MDP), N-acetyl-nor-muramyl-L-alanyl- D-isoglutamine (CGP 11637, referred to as nor-MDP), N- acetylmuramyl-L-alanyl-D-isoglutaminyl-L-alanine-2-(1- 2-dipalmitoyl -sn-glycero-3-hydroxyphosphoryloxy)- ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion. The effectiveness of an adjuvant may be determined by measuring the amount of antibodies directed against an immunogenic peptide containing an HCV antigenic sequence resulting from administration of this peptide in vaccines which are also comprised of the various adjuvants.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0/5% to 10%, preferably 1%-2%. Oral formulations include such normally employed excipients as, for example, pharmaceutical grades of mannitol, lactose, starch, magnesium stearate, sodium saccharine, cellulose, magnesium carbonate, and the like.

The examples below are provided for illustrative purposes and are not intended to limit the scope of the present invention.

### I. Detection of HCV RNA from Serum

RNA was extracted from serum using guanidinium salt, phenol and chloroform according to the instructions of the kit manufacturer (RNAzol™ B kit, Cinna/Biotecx). Extracted RNA was precipitated with isopropanol and washed with ethanol. A total of 25 µl serum was processed for RNA isolation, and the purified RNA was resuspended in 5 µl diethyl pyrocarbonate treated water for subsequent cDNA synthesis.

### II. cDNA Synthesis and Polymerase Chain Reaction (PCR) Amplification

Table 1 lists the sequence and position (with reference to HCV1) of all the PCR primers and probes used in these examples. Letter designations for nucleotides are consistent with 37 C.F.R. §§1.821-1.825. Thus, the letters A, C, G, T, and U are used in the ordinary sense of adenine, cytosine, guanine, thymine, and uracil. The letter M means A or C; R means A or G; W means A or T/U; S means C or G; Y means C or T/U; K means G or T/U; V means A or C or G, not T/U; H means A or C or T/U, not G; D means A or G or T/U, not C; B means C or G or T/U, not A; N means (A or C or G or T/U) or (unknown or other). Table 1 is set forth below:

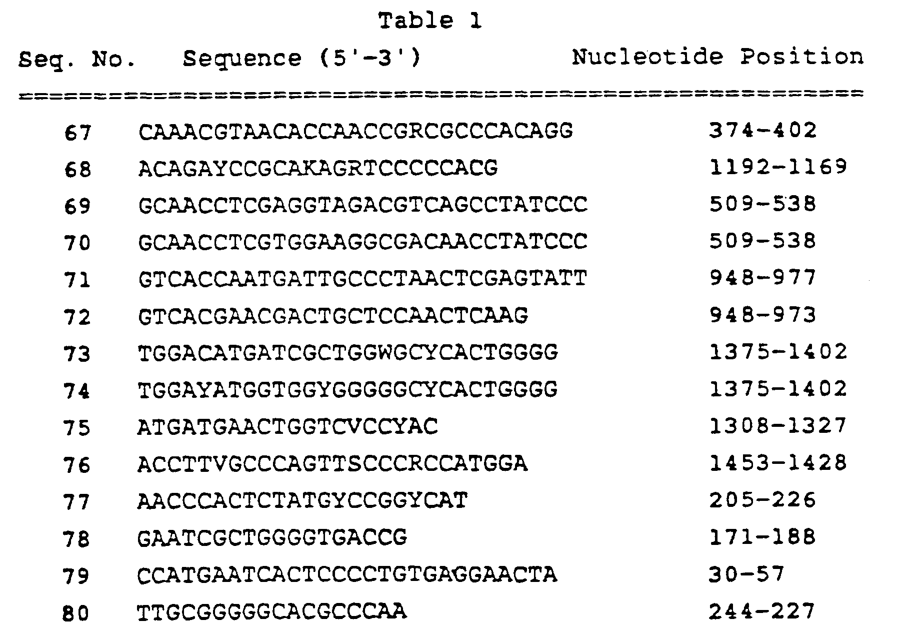

For cDNA synthesis and PCR amplification, a protocol developed by Perkin-Elmer/Cetus (GeneAmp® RNA PCR kit) was used. Both random hexamer and primers with specific complementary sequences to HCV were employed to prime the reverse transcription (RT) reaction. All processes, except for adding and mixing reaction components, were performed in a thermal cycler (MJ Research, Inc.). The first strand cDNA synthesis reaction was inactivated at 99°C for 5 min, and then cooled at 50°C for 5 min before adding reaction components for subsequent amplification. After an initial 5 cycles of 97°C for 1 min, 50°C for 2 min, and 72°C for 3 min, 30 cycles of 94°C for 1 min, 55°C for 2 min, and 72°C for 3 min followed, and then a final 7 min of elongation at 72°C.

For the genotyping analysis, sequences 67 and 68 were used as primers in the PCR reaction. These primers amplify a segment corresponding to the core and envelope regions. After amplification, the reaction products were separated on an agarose gel and then transferred to a nylon membrane. The immobilized reaction products were allowed to hybridize with a ³²P-labelled nucleic acid corresponding to either Genotype I (core or envelope 1) or Genotype II (core or envelope 1). Nucleic acid corresponding to Genotype 1 comprised sequences numbered 69 (core), 71 (envelope), and 73 (envelope). Nucleic acid corresponding to Genotype II comprised sequences numbered 70 (core), 72 (envelope), and 74 (envelope).

The Genotype I probes only hybridized to the product amplified from isolates which had Genotype I sequence. Similarly, Genotype II probes only hybridized to the product amplified from isolates which had Genotype II sequence.

In another experiment, PCR products were generated using sequences 79 and 80. The products were analyzed as described above except Sequence No. 73 was used to detect Genotype I, Sequence No. 74 was used to detect Genotype II, Sequence No. 77 (5'UT) was used to detect Genotype III, and Sequence No. 78 (5'UT) was used to detect Genotype IV. Each sequence hybridized in a genotype specific manner.

### III. Detection of HCV GI-GIV using a sandwich hybridization assay for HCV RNA

An amplified solution phase nucleic acid sandwich hybridization assay format is described in this example. The assay format employs several nucleic acid probes to effect capture and detection. A capture probe nucleic acid is capable of associating a complementary probe bound to a solid support and HCV nucleic acid to effect capture. A detection probe nucleic acid has a first segment (A) that binds to HCV nucleic acid and a second segment (B) that hybridizes to a second amplifier nucleic acid. The amplifier nucleic acid has a first segment (B*) that hybridizes to segment (B) of the probe nucleic acid and also comprises fifteen iterations of a segment (C). Segment C of the amplifier nucleic acid is capable of hybridizing to three labeled nucleic acids.

Nucleic acid sequences which correspond to nucleotide sequences of the envelope 1 gene of Group I HCV isolates are set forth in sequences numbered 81-99. Table 2 sets forth the area of the HCV genome to which the nucleic acid sequences correspond and a preferred use of the sequences.

**Table 2**

| Probe Type | Sequence No. | Complement of Nucleotide Numbers |
|---|---|---|
| Label | 81 | 879-911 |
| Label | 82 | 912-944 |
| Capture | 83 | 945-977 |
| Label | 84 | 978-1010 |
| Label | 85 | 1011-1043 |
| Label | 86 | 1044-1076 |
| Label | 87 | 1077-1109 |
| Capture | 88 | 1110-1142 |
| Label | 89 | 1143-1175 |
| Label | 90 | 1176-1208 |
| Label | 91 | 1209-1241 |
| Label | 92 | 1242=1274 |
| Capture | 93 | 1275-1307 |
| Label | 94 | 1308-1340 |
| Label | 95 | 1341-1373 |
| Label | 96 | 1374-1406 |
| Label | 97 | 1407-1439 |
| Capture | 98 | 1440-1472 |
| Label | 99 | 1473-1505 |

Nucleic acid sequences which correspond to nucleotide sequences of the envelope 1 gene of Group II HCV isolates are set forth in sequences 100-118. Table 3 sets forth the area of the HCV genome to which the nucleic acid corresponds and the preferred use of the sequences.

**Table 3**

| Probe Type | Sequence No. | Complement of Nucleotide Numbers |
|---|---|---|
| Label | 100 | 879-911 |
| Label | 101 | 912-944 |
| Capture | 102 | 945-977 |
| Label | 103 | 97B-1010 |
| Label | 104 | 1011-1043 |
| Label | 105 | 1044-1076 |
| Label | 106 | 1077-1109 |
| Capture | 107 | 1110-1142 |
| Label | 108 | 1143-1175 |
| Label | 109 | 1176-1208 |
| Label | 110 | 1209-1241 |
| Label | 111 | 1242=1274 |
| Capture | 112 | 1275-1307 |
| Label | 113 | 1308-1340 |
| Label | 114 | 1341-1373 |
| Label | 115 | 1374-1406 |
| Label | 116 | 1407-1439 |
| Capture | 117 | 1440-1472 |
| Label | 118 | 1473-1505 |

Nucleic acid sequences which correspond to nucleotide sequences in the C gene and the 5'UT region are set forth in sequences 119-145. Table 4 identifies the sequence with a preferred use.

**Table 4**

| Probe Type | Sequence No. |
|---|---|
| Capture | 119 |
| Label | 120 |
| Label | 121 |
| Label | 122 |
| Capture | 123 |
| Label | 124 |
| Label | 125 |
| Label | 126 |
| Capture | 127 |
| Label | 128 |
| Label | 129 |
| Label | 130 |
| Capture | 131 |
| Label | 132 |
| Label | 133 |
| Label | 134 |
| Label | 135 |
| Capture | 136 |
| Label | 137 |
| Label | 138 |
| Label | 139 |
| Capture | 140 |
| Label | 141 |
| Label | 142 |
| Label | 143 |
| Capture | 144 |
| Label | 145 |

The detection and capture probe HCV-specific segments, and their respective names as used in this assay were as follows.
Capture sequences are sequences numbered 119-122 and 141-144.
Detection sequences are sequences numbered 119-140.

Each detection sequence contained, in addition to the sequences substantially complementary to the HCV sequences, a 5' extension (B) which extension (B) is complementary to a segment of the second amplifier nucleic acid. The extension (B) sequence is identified in the Sequence Listing as Sequence No. 146, and is reproduced below.

AGGCATAGGACCCGTGTCTT

Each capture sequence contained, in addition to the sequences substantially complementary to HCV sequences, a sequence complementary to DNA bound to a solid phase. The sequence complementary to DNA bound to a solid support was carried downstream from the capture sequence. The sequence complementary to the DNA bound to the support is set forth as Sequence No. 147 and is reproduced below.

CTTCTTTGGAGAAAGTGGTG

Microtiter plates were prepared as follows. White Microlite 1 Removawell strips (polystyrene microtiter plates, 96 wells/plate) were purchased from Dynatech Inc.

Each well was filled with 200 µl 1 N HCl and incubated at room temperature for 15-20 min. The plates were then washed 4 times with 1X PBS and the wells aspirated to remove liquid. The wells were then filled with 200 µl 1 N NaOH and incubated at room temperature for 15-20 min. The plates were again washed 4 times with 1X PBS and the wells aspirated to remove liquid.

Poly(phe-lys) was purchased from Sigma Chemicals, Inc. This polypeptide has a 1:1 molar ratio of phe:lys and an average m.w. of 47,900 gm/mole. It has an average length of 309 amino acids and contains 155 amines/mole. A 1 mg/ml solution of the polypeptide was mixed with 2M NaCl/1X PBS to a final concentration of 0.1 mg/ml (pH 6.0). A volume of 200 µl of this solution was added to each well. The plate was wrapped in plastic to prevent drying and incubated at 30°C overnight. The plate was then washed 4 times with 1X PBS and the wells aspirated to remove liquid.

The following procedure was used to couple the nucleic acid, a complementary sequence to Sequence No. 147, to the plates, hereinafter referred to as immobilized nucleic acid. Synthesis of immobilized nucleic acid having a sequence complementary to Sequence No. 133 was described in EPA 883096976. A quantity of 20 mg disuccinimidyl suberate was dissolved in 300 µl dimethyl formamide (DMF). A quantity of 26 OD₂₆₀ units of immobilized nucleic acid was added to 100 µl coupling buffer (50 mM sodium phosphate, pH 7.8). The coupling mixture was then added to the DSS-DMF solution and stirred with a magnetic stirrer for 30 min. An NAP-25 column was equilibrated with 10 mM sodium phosphate, pH 6.5. The coupling mixture DSS-DMF solution was added to 2 ml 10 mM sodium phosphate, pH 6.5, at 4°C. The mixture was vortexed to mix and loaded onto the equilibrated NAP-25 column. DSS-activated immobilized nucleic acid DNA was eluted from the column with 3.5 ml 10 mM sodium phosphate, pH 6.5. A quantity of 5.6 OD₂₆₀ units of eluted DSS-activated immobilized nucleic acid DNA was added to 1500 ml 50 mM sodium phosphate, pH 7.8. A volume of 50 µl of this solution was added to each well and the plates were incubated overnight. The plate was then washed 4 times with 1X PBS and the wells aspirated to remove liquid.

Final stripping of plates was accomplished as follows. A volume of 200 µl of 0.2N NaOH containing 0.5% (w/v) SDS was added to each well. The plate was wrapped in plastic and incubated at 65°C for 60 min. The plate was then washed 4 times with 1X PBS and the wells aspirated to remove liquid. The stripped plate was stored with desiccant beads at 2-8°C.

Serum samples to be assayed were analyzed using PCR followed by sequence analysis to determine the genotype.

Sample preparation consisted of delivering 50 µl of the serum sample and 150 µl P-K Buffer (2 mg/ml proteinase K in 53 mM Tris-HCl, pH 8.0/0.6 M NaCl/0.06 M sodium citrate/8 mM EDTA, pH 8.0/1.3%SDS/16µg/ml sonicated salmon sperm DNA/7% formamide/50 fmoles capture probes/160 fmoles detection probes) to each well. Plates were agitated to mix the contents in the well, covered and incubated for 16 hr at 62°C.

After a further 10 minute period at room temperature, the contents of each well were aspirated to remove all fluid, and the wells washed 2X with washing buffer (0.1% SDS/0.015 M NaCl/ 0.0015 M sodium citrate). The amplifier nucleic acid was then added to each well (50 µl of 0.7 fmole/µl solution in 0..48 M NaCl/0.048 M sodium citrate/0.1% SDS/0.5% "blocking reagent" (Boehringer Mannheim, catalog No. 1096 176)). After covering the plates and agitating to mix the contents in the wells, the plates were incubated for 30 min. at 52°C.

After a further 10 min period at room temperature, the wells were washed as described above.

Alkaline phosphatase label nucleic acid, disclosed in EP 883096976, was then added to each well (50 µl/well of 2.66 fmoles/µl). After incubation at 52°C for 15 min., and 10 min. at room temperature, the wells were washed twice as above and then 3X with 0.015 M NaCl/0.0015 M sodium citrate.

An enzyme-triggered dioxetane (Schaap et al., Tet. Lett. (1987) 28:1159-1162 and EPA Pub. No. 0254051), obtained from Lumigen, Inc., was employed. A quantity of 50 µl Lumiphos 530 (Lumigen) was added to each well. The wells were tapped lightly so that the reagent would fall to the bottom and gently swirled to distribute the reagent evenly over the bottom. The wells were covered and incubated at 37°C for 20-40 min.

Plates were then read on a Dynatech ML 1000 luminometer. Output was given as the full integral of the light produced during the reaction.

The assay positively detected each of the serum samples, regardless of genotype.

### IV. Expression of the Polypeptide Encoded in Sequences Defined by Differing Genotypes

HCV polypeptides encoded by a sequence within sequences 1-66 are expressed as a fusion polypeptide with superoxide dismutase (SOD). A cDNA carrying such sequences is subcloned into the expression vector pSODcfl (Steimer et al. 1986)).

First, DNA isolated from pSODcfl is treated with BamHI and EcoRI, and the following linker was ligated into the linear DNA created by the restriction enzymes: 
5    GAT CCT GGA ATT CTG ATA AGA
         CCT TAA GAC TAT TTT AA    3
 After cloning, the plasmid containing the insert is isolated.

Plasmid containing the insert is restricted with EcoRI. The HCV cDNA is ligated into this EcoRI linearized plasmid DNA. The DNA mixture is used to transform E. coli strain D1210 (Sadler et al. (1980)). Polypeptides are isolated on gels.

### V. Antigenicity of Polypeptides

The antigenicity of polypeptides formed in Section IV is evaluated in the following manner. Polyethylene pins arranged on a block in an 8 12 array (Coselco Mimetopes, Victoria, Australia) are prepared by placing the pins in a bath (20% v/v piperidine in dimethylformamide (DMF)) for 30 minutes at room temperature. The pins are removed, washed in DMF for 5 minutes, then washed in methanol four times (2 min/wash). The pins are allowed to air dry for at least 10 minutes, then washed a final time in DMF (5Min). 1-Hydroxybenzotriazole (HOBt, 367 mg) is dissolved in DMF (80 µL) for use in coupling Fmoc-protected polypeptides prepared in Section IV.

The protected amino acids are placed in micro-titer plate wells with HOBt, and the pin block placed over the plate, immersing the pins in the wells. The assembly is then sealed in a plastic bag and allowed to react at 25°C for 18 hours to couple the first amino acids to the pins. The block is then removed, and the pins washed with DMF (2 min.), MeOH (4 x, 2 min.), and again with DMF (2 min.) to clean and deprotect the bound amino acids. The procedure is repeated for each additional amino acid coupled, until all octamers are prepared.

The free N-termini are then acetylated to compensate for the free amide, as most of the epitopes are not found at the N-terminus and thus would not have the associated positive charge. Acetylation is accomplished by filling the wells of a microtiter plate with DMF/acetic anhydride/triethylamine (5:2:1 v/v/v) and allowing the pins to react in the wells for 90 minutes at 20°C. The pins are then washed with DMF (2 min.) and MeOH (4 x, 2 min.), and air dried for at least 10 minutes.

The side chain protecting groups are removed by treating the pins with trifluoroacetic acid/phenol/dithioethane (95:2.5:1.5, v/v/v) in polypropylene bags for 4 hours at room temperature. The pins are then washed in dichloromethane (2 x, 2 min.), 5% di-isopropylethylamine/dichloromethane (2 x, 5 min.), dichloromethane. (5 min.), and air-dried for at least 10 minutes. The pins are then washed in water (2 min.), MeOH (18 hours), dried in vacuo, and stored in sealed plastic bags over silica gel. IV.B.15.b Assay of Peptides.

Octamer-bearing pins are treated by sonicating for 30 minutes in a disruption buffer (1% sodium dodecylsulfate, 0.1% 2-mercaptoethanol, 0.1 M NaH2PO4) at 60°C. The pins are then immersed several times in water (60°C), followed by boiling MeOH (2 min.), and allowed to air dry.

The pins are then precoated for 1 hour at 25°C in microtiter wells containing 200 µL blocking buffer (1% ovalbumin, 1% BSA, 0.1% Tween, and 0.05% NaN3 in PBS), with agitation. The pins are then immersed in microtiter wells containing 175 µL antisera obtained from human patients diagnosed as having HCV and allowed to incubate at 4°C overnight. The formation of a complex between polyclonal antibodies of the serum and the polypeptide initiates that the peptides give rise to an immune response in vivo. Such peptides are candidates for the development of vaccines.

Thus, this invention has been described and illustrated. It will be apparent to those skilled in the art that many variations and modifications can be made without departing from the purview of the appended claims and without departing from the teaching and scope of the present invention.

## Claims

1. As a composition of matter, a non-naturally occurring nucleic acid having a non-HCV-1 nucleotide sequence of eight or more nucleotides corresponding to a nucleotide sequence within the hepatitis C virus genome, other than a sequence of the NS5 region of HCV-K1, HCV-K2a and HCV-K2b and other than a sequence of the core region, envelope 1 region, 5' UT region, NS3 region, NS4 region and NS5 region of HCV-J1 and HCV-J4.

2. The composition of claim 1 wherein said nucleotide sequence corresponding to a non-HCV-1 nucleotide sequence within the hepatitis C virus genome is selected from the regions consisting of the NS5 region, envelope 1 region, 5'UT region, and the core region.

3. The composition of claim 1 wherein said nucleotide sequence corresponding to a non-HCV-1 nucleotide sequence within the hepatitis C virus genome corresponds to a sequence in the NS5 region.

4. The composition of claim 3 wherein said nucleotide sequence corresponding to a non-HCV-1 sequence within the hepatitis C virus genome is selected from a sequence within sequences numbered 2, 4-6, 10-12, 14-16, 18-22.

5. The composition of claim 1 wherein said nucleotide sequence corresponding to a non-HCV-1 nucleotide sequence within the hepatitis C virus genome corresponds to a sequence in the envelope 1 region.

6. The composition of claim 5 wherein said nucleotide sequence corresponding to a non-HCV-1 sequence within the hepatitis C virus genome corresponds to a sequence within sequence numbers 24-32.

7. The composition of claim 1 wherein at least one sequence corresponding to a non-HCV-1 nucleotide sequence within the hepatitis C virus genome corresponds to a sequence in the 5'UT region.

8. The composition of claim 7 wherein said nucleotide sequence corresponding to a non-HCV-1 sequence within the hepatitis C virus genome corresponds to a sequence within sequences numbered 34-39 and 41-51.

9. The composition of claim 1 wherein said nucleotide sequence corresponding to a non-HCV-1 nucleotide sequence within the hepatitis C virus genome corresponds to a sequence in the core region.

10. The composition of claim 9 wherein said nucleotide sequence corresponding to a non-HCV-1 sequence within the hepatitis C virus genome corresponds to a within sequences numbered 52-57 and 58-66.

11. The composition of claim 1 wherein said non-naturally occurring nucleic acid has a nucleotide sequence corresponding to one or more genotypes of hepatitis C virus.

12. The composition of claim 11 wherein said non-naturally occurring nucleic acid has a sequence corresponding to a sequence of a first genotype which first genotype is defined by sequences numbered 1-6 in the NS5 region, 23-25 in the envelope 1 region, 33-38 in the 5'UT region, and 52-57 in the core region.

13. The composition of claim 11 wherein said non-naturally occurring nucleic acid has a sequence corresponding to a sequence of a second genotype which second genotype is defined by sequences numbered 7-12 in the NS5 region, 26-28 in the envelope 1 region, 39-45 in the 5'UT region, and 58-64 in the core region.

14. The composition of claim 11 wherein said non-naturally occurring nucleic acid has a sequence corresponding to a sequence of a third genotype which third genotype is defined by sequences numbered 13-17 in the NS5 region, 32 in the envelope 1 region, 46-47 in the 5'UT region and 65-66 in the core region.

15. The composition of claim 11 wherein said non-naturally occurring nucleic acid has a sequence corresponding to a sequence of a fourth genotype which fourth genotype is defined by sequences numbered 20-22 in the NS5 region, 29-31 in the envelope 1 region and 48-49 in the 5'UT region.

16. The composition of claim 11 wherein said non-naturally occurring nucleic acid has a sequence corresponding to a sequence of a fifth genotype which fifth genotype is defined by sequences numbered 18-19 in the NS5 region and 50-51 in the 5'UT region.

17. The composition of any one of claims 1 to 16 wherein said non-naturally occurring nucleic acid is capable of priming a reaction for the synthesis of nucleic acid to form a nucleic acid having a nucleotide sequence corresponding to hepatitis C virus.

18. The composition of any one of claims 1 to 16 wherein said non-naturally occurring nucleic acid has label means for detecting a hybridization product.

19. The composition of any one of claims 1 to 16 wherein said non-naturally occurring nucleic acid has support means for separating a hybridization product from solution.

20. The composition of any one of claims 1 to 16 wherein said non-naturally occurring nucleic acid prevents the transcription or translation of viral nucleic acid.

21. A composition according to any one of claims 1 to 6 or 9 to 16 wherein said non-HCV-1 nucleotide sequence is at least nine nucleotides, with the proviso that said sequence is not from a 5' UT region.

22. A method of forming a hybridization product with a hepatitis C virus nucleic acid comprising the following steps:
a) placing a non-naturally occurring nucleic acid having a non-HCV-1 nucleotide sequence or eight or more nucleotides corresponding to a sequence in the hepatitis C viral genome, other than a sequence of the NS5 region of HCV-K1, HCV-K2a and HCV-K2b and other than a sequence of the core region, envelope 1 region, 5' UT region, NS3 region, NS4 region and NS5 region, of HCV-J1 and HCV-J4, into conditions in which hybridization conditions can be imposed, said non-naturally occurring nucleic acid capable of forming a hybridization product with said hepatitis C virus nucleic acid under hybridization conditions; and
b) imposing hybridization conditions to form a hybridization product in the presence of hepatitis C virus nucleic acid.

23. A method according to claim 22 wherein said nucleotide sequence is as defined in any one of claims 2 to 20.

24. As a composition of matter, a non-naturally occurring polypeptide encoded by a non-HCV-1 nucleotide sequence of nine or more nucleotides which sequence of nine or more nucleotides corresponds to a sequence within hepatitis C virus genomic sequences, other than a sequence of the NS5 region of HCV-K1, HCV-K2a and HCV-K2b and other than a sequence of the core region, envelope 1 region, 5' UT region, NS3 region, NS4 region and NS5 region, of HCV-J1 and HCV-J4.

25. A composition according to claim 24 wherein said non-HCV-1 sequence is as defined in claim 21.

26. The composition of claim 24 or 25 wherein said polypeptide is capable of generating an immune reaction in a host.

27. An antibody capable of selectively binding to the composition of claim 24 or 25.

28. A method of detecting one or more genotypes of hepatitis C virus comprising the following steps:
a) placing a non-naturally occurring nucleic acid having a non-HCV-1 nucleotide sequence of eight or more nucleotides as defined in any one of claims 1 to 16 under conditions where hybridization conditions can be imposed;
b) imposing hybridization conditions to form a hybridization product in the presence of hepatitis C virus nucleic acid; and
c) monitoring the non-naturally occurring nucleic acid for the formation of a hybridization product, which hybridization product is indicative of the presence of the genotype of hepatitis C virus.

29. The method of claim 28 wherein said non-naturally occurring nucleic acid has a sequence corresponding to a sequence numbered 67-145.

30. The method of claim 29 wherein said non-naturally occurring nucleic acid has a sequence corresponding to a sequence numbered 69, 71, 73 and 81-99 to identify Group I genotypes in the core and region of the HCV genome.

31. The method of claim 29 wherein said non-naturally occurring nucleic acid has a sequence corresponding to a sequence numbered 70, 72, 70 and 100-118 to identify Group II genotypes in the core and envelope regions of the HCV genome.

32. The method of claim 29 wherein said non-naturally occurring nucleic acid has a sequence corresponding to a sequence numbered 77 to identify Group III genotypes in the 5' UT region of the HCV genome.

33. The method of claim 29 wherein said non-naturally occurring nucleic acid has a sequence numbered 79 to identify Group IV genotypes in the 5' UT region of the HCV genome.
